# EUROPEAN PATENT SPECIFICATION

(11) **EP 2 886 068 B1**
(45) Date of publication and mention of the grant of the patent: **18.07.2018**
(21) Application number: 14200121.3
(22) Date of filing: 23.12.2014
(51) Int. Cl.: A61B 17/072, A61B 17/29

(54) **SURGICAL INSTRUMENTS WITH ARTICULATABLE SHAFT ARRANGEMENTS**
CHIRURGISCHE INSTRUMENTE MIT BEWEGLICHEN SCHAFTANORDNUNGEN
INSTRUMENTS CHIRURGICAUX AVEC AGENCEMENTS D'ARBRE ARTICULABLES

(30) Priority: 23.12.2013 US 201314138554
(43) Date of publication of application: 24.06.2015
(73) Proprietor: Ethicon LLC, 00969 Guaynabo (PR)
(72) Inventor: Baxter, III, Chester O, Loveland, OH Ohio 45140 (US); Shelton, IV, Frederick E., Hillsboro, OH Ohio 45133 (US)
(74) Representative: Carpmaels & Ransford LLP

(56) References cited:
- US-A- 5 329 923
- US-A- 5 673 841
- US-A- 5 704 534
- US-A1- 2004 034 343
- US-A1- 2013 334 281
- US-E1- R E38 335

## Description

### BACKGROUND

The present invention relates to surgical instruments and, in various embodiments, to surgical cutting and stapling instruments and staple cartridges therefor that are designed to cut and staple tissue.

US 2004/0034343 A1 discloses an articulating device for use with a medical insertion instrument comprising in part a first tube having a plurality of ribs defining a plurality of bending segments, a second tube axially disposed within the first tube, and means for transmitting an axial deflecting pull load. US 5,673,841 discloses a surgical instrument comprising a surgical end effector, and an elongated shaft assembly defining a longitudinal tool axis and operably coupled to the end effector, the elongated shaft assembly includes a flexible segment comprising a longitudinally extending elongated articular spine including two lateral sides, and a plurality of ribs extending laterally from each side along a length thereof.

US 5329923 discloses a torquable tubular member for use in a flexible elongate device comprising an elongate tubular body having a cylindrical wall and a longitudinal axis extending longitudinally of the cylindrical wall. The cylindrical wall has formed therein a plurality of spaced-apart slots spaced longitudinally of the longitudinal axis. In order to provide different degrees of flexibility in the tip section, the depths of the slots can be varied so as the slots become deeper, the backbone or rib becomes narrower to permit greater flexibility in the backbone or rib, and conversely if the slots are shallower, the backbone or rib will become wider to provide lesser flexibility. Thus the backbone or rib is tapered in a direction towards the distal extremity to provide gradually increasing flexibility toward the distal extremity. The present invention is defined by the features of the independent claim. Preferred embodiments are given in the dependent claims.

### BRIEF DESCRIPTION OF THE DRAWINGS

The features and advantages of various forms of the invention, and the manner of attaining them, will become more apparent and the invention itself will be better understood by reference to the following description of embodiments of the invention taken in conjunction with the accompanying drawings, wherein:
FIG. 1 is a perspective view of one form of a surgical instrument of the present invention;
FIG. 2 is an exploded perspective view of one form of surgical end effector of the present invention;
FIG. 3 is an exploded perspective view of a portion of the surgical instrument of FIG. 1;
FIG. 4 is an exploded perspective assembly view of another portion of the surgical instrument of FIG. 1;
FIG. 5 is an exploded perspective assembly view of a portion of the elongated shaft assembly of the surgical instrument of FIG. 1;
FIG. 6 is a perspective view of the surgical end effector of FIG. 2 and a distal closure tube segment;
FIG. 7 is a rear perspective view of a portion of an anvil embodiment;
FIG. 7A is an exploded perspective assembly view of another surgical end effector assembly;
FIG. 7B is a rear perspective view of a portion of another anvil assembly embodiment and another closure tube segment embodiment;
FIG. 7C is a perspective view of a portion of another anvil assembly and another distal closure tube segment;
FIG. 7D is an exploded perspective assembly view of another surgical end effector embodiment;
FIG. 7E is an exploded perspective assembly view of another surgical end effector embodiment;
FIG. 8 is a side cross-sectional view of a surgical end effector and distal closure tube segment with the anvil assembly in an open position;
FIG. 9 is another side cross-sectional view of the surgical end effector and distal closure tube segment of FIG. 8;
FIG. 10 is a perspective view of a portion of the surgical instrument of FIG. 1 with a portion of the handle housing removed;
FIG. 11 is a perspective view of a portion of a firing drive system;
FIG. 12 is a perspective view of an intermediate portion of an elongated shaft assembly example not falling under the scope of the invention;
FIG. 13 is an elevational view of the distal end of the intermediate shaft portion of FIG. 12;
FIG. 14 is side elevational view of the intermediate shaft portion of FIGS. 12 and 13;
FIG. 15 is a plan view of the intermediate shaft portion of FIGS. 12-14;
FIG. 16 is an enlarged side elevational view of portions of adjacent ribs of the intermediate shaft portion of FIGS. 12-15;
FIG. 17 is a plan view of an intermediate shaft portion embodiment according to the invention;
FIG. 18 is a side elevational view of the intermediate shaft portion of FIG. 17; and
FIG. 19 is a cross-sectional plan view of the intermediate shaft portion of FIGS. 17 and 18 articulated into a substantial U-shape.

Corresponding reference characters indicate corresponding parts throughout the several views. The exemplifications set out herein illustrate certain embodiments of the invention, in one form, and such exemplifications are not to be construed as limiting the scope of the invention in any manner.

### DETAILED DESCRIPTION

Exemplary embodiments and examples not falling under the scope of the invention will now be described. It will be understood and it is intended that where certain characteristics or attributes are described in connection with one embodiment or example, those characteristics or attributes may also be incorporated into any other embodiment or example described with which they are compatible.

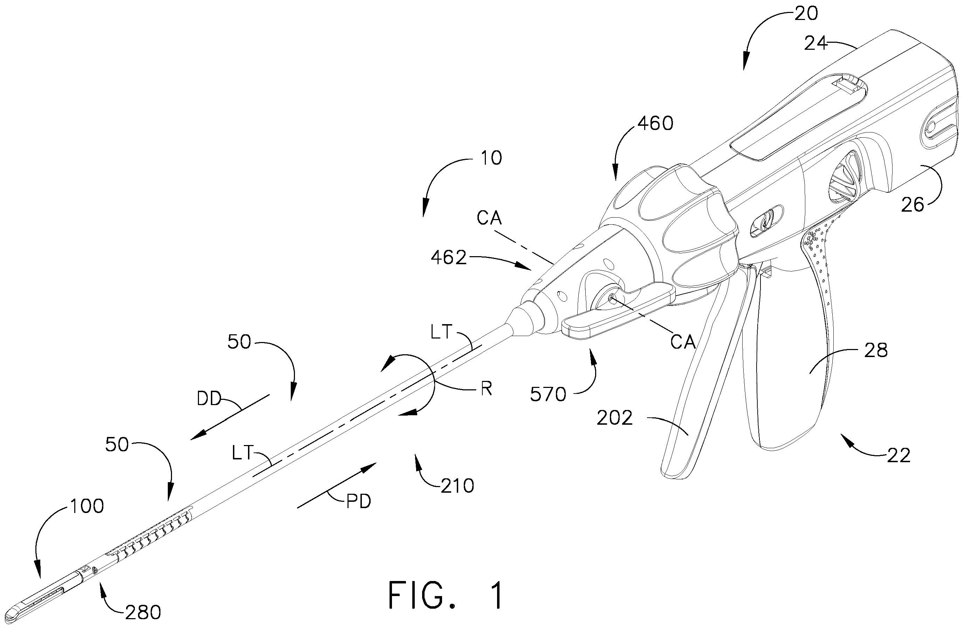

FIG. 1 illustrates an exemplary surgical instrument 10 which includes a housing 20, an elongated shaft assembly 50 that operably protrudes from the housing 20 and which is operably coupled to a surgical end effector 100. The surgical instrument 10 depicted in the FIG. 1 comprises a housing 20 that consists of a handle 22 that is configured to be grasped, manipulated and actuated by a clinician. As the present Detailed Description proceeds, however, it will be understood that the various unique and novel arrangements of the shaft arrangements and end effector arrangements disclosed herein also may be effectively employed in connection with robotically-controlled surgical systems. Thus, the term "housing" also encompass a housing or similar portion of a robotic system that houses or otherwise operably supports at least one drive system that is configured to generate and apply at least one control motion which could be used to actuate surgical end effectors attached thereto. The term "frame" refers to a portion of a handheld surgical instrument. The term "frame" also represents a portion of a robotically controlled surgical instrument and/or a portion of the robotic system that is used to operably control a surgical instrument. For example, U.S. Patent Application Publication No. 2014/0001235 A1, entitled ROBOTICALLY-POWERED SURGICAL DEVICE WITH MANUALLY ACTUATABLE REVERSING SYSTEM, filed June 28, 2012 discloses various robotic system arrangements that may also be effectively employed. Furthermore, as will be discussed in further detail below, the surgical instrument 10 depicted in at least some of the accompanying drawings employs a motor for generating control motions for actuating various components and features of the surgical end effector. As the present Detailed Description proceeds, however, those of ordinary skill in the art will appreciate that certain features and advantages of the present invention also are effectively attained in connection with surgical instruments that are equipped with manually generated (i.e., non-motor generated) actuation and control motions.

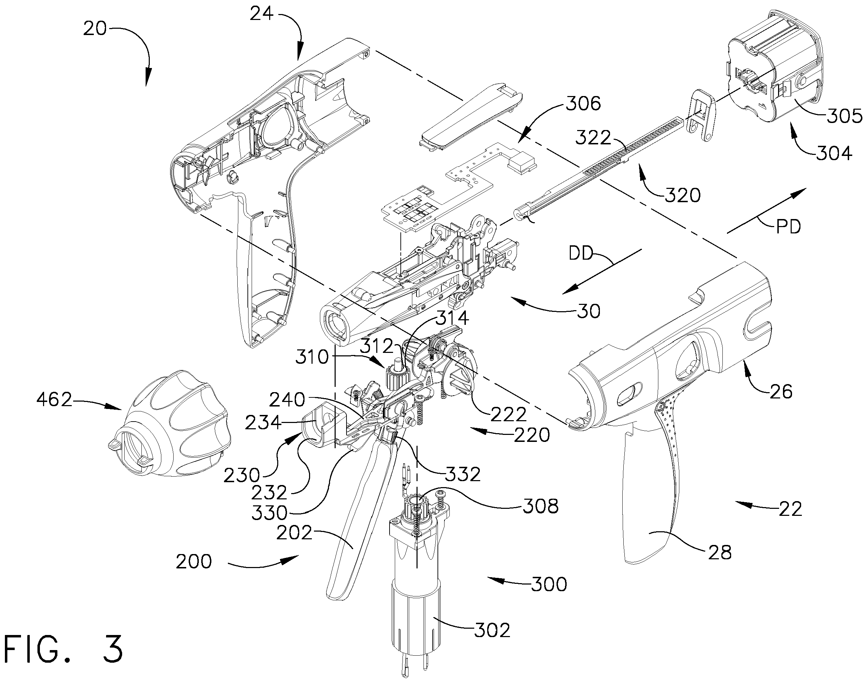

As illustrated in FIGS. 1 and 3, the handle 22 comprises a pair of interconnectable housing segments 24, 26 that are interconnected by screws, snap features, adhesive, etc. As used herein, the term "snap feature" includes, but is not limited to, for example, a tab that has a protrusion thereon that is configured to retainingly engage a corresponding mating portion of another component. Such features may be designed to releasably engage the mating portion or may not be designed or intended to be removed, as the case may be . In the illustrated arrangement, the handle housing segments 24, 26 cooperate to form a pistol grip portion 28 that is gripped and manipulated by the clinician. As will be discussed in further detail below, the handle 22 operably supports a plurality of drive systems or control systems therein that are configured to generate and apply various control motions to corresponding component portions of the elongated shaft assembly 50 that is operably attached to the surgical end effector 100. The surgical end effector 100 is configured to cut and fasten tissue, for example.

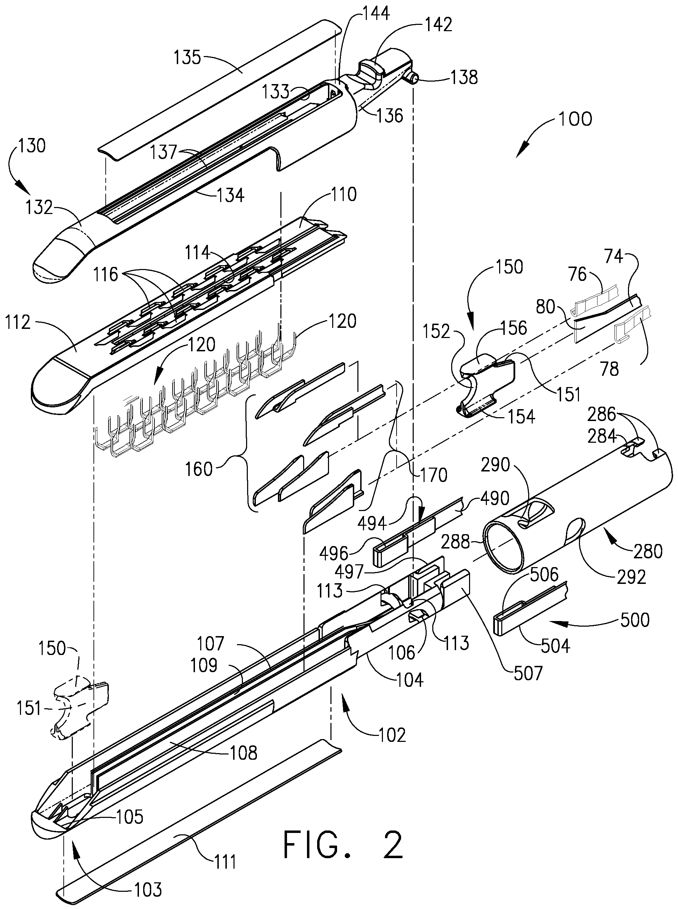

FIG. 2 illustrates one form of surgical end effector 100 that is employed. As can be seen in that Figure, the surgical end effector 100 comprises an elongated channel 102 that is configured to receive a surgical fastener cartridge 110 therein. The surgical fastener cartridge 110 includes a cartridge body 112 that has a centrally disposed elongated slot 114 therein. The cartridge body 112 further include rows of fastener pockets 116 that are located on each side of the elongated slot 114 and which are configured to support corresponding surgical fasteners 120 therein. The elongated channel 102 further operably support a tissue-cutting member or knife assembly 150 therein that is configured to axially travel in the slot 114 in the cartridge body 112 when installed in the elongate channel 102. The knife assembly 150 is configured with a tissue cutting edge 152 that is centrally disposed between a lower foot 154 and an upper foot or tab 156. As will be discussed in further detail below, the knife assembly 150 is configured to be axially driven within the elongated channel 102 and the surgical fastener cartridge 110 in response to motions applied thereto by a firing drive system 300.

As may also be seen in FIG. 2, the surgical end effector 100 further include an anvil assembly 130 that is movably supported on the elongate channel 102. The anvil assembly 130 is movable relative to the surgical fastener cartridge 110, for example, in response to closing and opening motions transferred thereto from a closure drive system 200. In other arrangements, however, the anvil assembly is fixed and the surgical fastener cartridge is configured to move relative to the anvil assembly upon application of closure motions thereto. In one arrangement, for example, the anvil assembly 130 includes an anvil body portion 132 that has a fastener forming surface 134 formed on the underside thereof. The fastener forming surface 134 comprises a series of forming pockets (not shown) that correspond to the surgical fasteners 120 supported in the surgical fastener cartridge 110. As the legs of the surgical fasteners 120 are driven into forming contact with the corresponding forming pockets in the anvil assembly 130, they are formed into a desired tissue-retaining configuration. The anvil assembly 130 further include an anvil mounting portion 136 that has a pair of trunnions 138 protruding therefrom that are received within corresponding arcuate slots 106 formed in a proximal mounting portion 104 of the elongated channel 102. In various arrangements, the surgical fasteners 120 are driven out of their respective fastener pockets 116 in the surgical fastener cartridge 110 by corresponding sled assemblies 160 and 170 that are movably supported within the elongated channel 102 and are movable in response to firing motions applied thereto by the firing drive system 300.

Referring now to FIG. 3, the handle 22 further include a frame 30 that operably supports various components of the closure drive system 200 and the firing drive system 300. The closure drive system 200 includes an actuator in the form of a closure trigger 202 that is pivotally supported by the frame 30. The closure trigger 202 is pivotally supported by frame 30 such that when the clinician grips the pistol grip portion 28 of the handle 22, the closure trigger 202 is easily pivoted from a starting or unactuated position to an actuated position and more particularly to a fully compressed or fully actuated position. The closure trigger 202 is biased into the unactuated position by spring or other biasing arrangement (not shown). Various details regarding the certain aspects of the construction and operation of the closure drive system 200 are found in U.S. Patent Application Publication No. 2014/0263542 A1, filed March 14, 2013, and entitled ARTICULATABLE SURGICAL INSTRUMENT COMPRISING A FIRING DRIVE. As discussed in that reference and as shown in FIG. 3 herein, the closure trigger 202 is configured to cooperate with a closure release assembly 220 that is pivotally coupled to the frame 30. The closure release assembly 220 comprises a release button assembly 222 that is pivoted in a counterclockwise direction by a release spring (not shown). As the clinician depresses the closure trigger 202 from its unactuated position towards the pistol grip portion 28 of the handle 22, the closure release assembly 220 serves to lock the closure trigger 202 in the fully actuated position. When the clinician desires to unlock the closure trigger 202 to permit it to be biased to the unactuated position, the clinician simply pivots the closure release button assembly 220 to cause it to disengage the closure trigger arrangement and thereby permit the closure trigger 202 to pivot back to the unactuated position. Other closure trigger locking and release arrangements may also be employed.

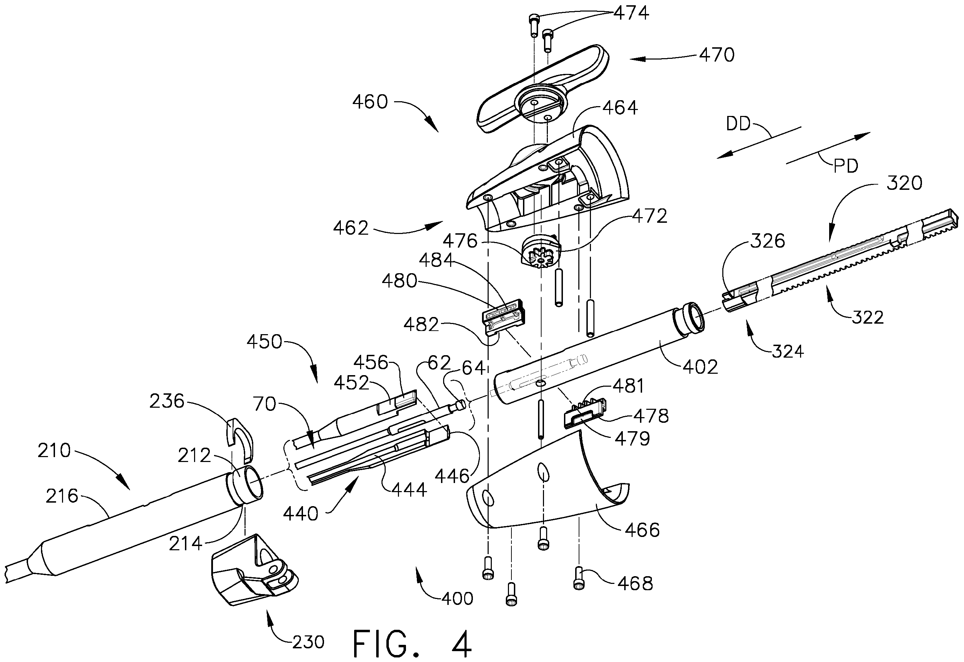

Referring to FIGS. 3 and 4, the closure drive system 200 further comprises a proximal closure tube segment 210 that has a proximal end 212 that is adapted to be rotatably coupled to a closure tube attachment yoke 230. The proximal end 212 of the proximal closure tube segment 210 is configured to be received within a cradle 232 (FIG. 3) in the closure tube attachment yoke 230 to permit relative rotation relative thereto. The proximal closure tube segment 210 is rotatably attached to the closure tube attachment yoke 230 by a U-shaped connector 236 that is configured to be received in an annular slot 214 in the proximal end 212 of the proximal closure tube segment 210 and is seated in a slot 234 (FIG. 3) in the closure tube attachment yoke 230. Such arrangement serves to rotatably couple the proximal closure tube segment 210 to the closure tube attachment yoke 230 such that the proximal closure tube segment 210 rotate relative thereto. More specifically, such arrangement facilitates manual rotation of the elongated shaft assembly 50 relative to the handle 22 about a longitudinal tool axis "LT-LT" defined by the elongated shaft assembly 50 to enable the clinician to rotate the surgical end effector 100 in the manner represented by arrow "R" in FIG. 1.

In various arrangements, the closure tube attachment yoke 230 is movably mounted on a proximal articulation tube 402 of an articulation system 400 which will be discussed in further detail below. Such arrangement permits the closure tube attachment yoke 230 to move axially on the proximal articulation tube 402 in response to actuation of the closure trigger 202. In particular, the closure tube attachment yoke 230 is pivotally coupled to the closure trigger 202 by a closure linkage bar 240. See FIG. 3. Thus, when the clinician pivots the closure trigger 202 inward toward the pistol grip portion 28 of the handle 22, the closure tube attachment yoke 230 will be advanced in the distal direction "D". When the firing trigger 202 is returned to the unactuated position, the closure tube attachment yoke 230 will be advanced proximally (direction "P") on the proximal articulation tube 402 to a starting position.

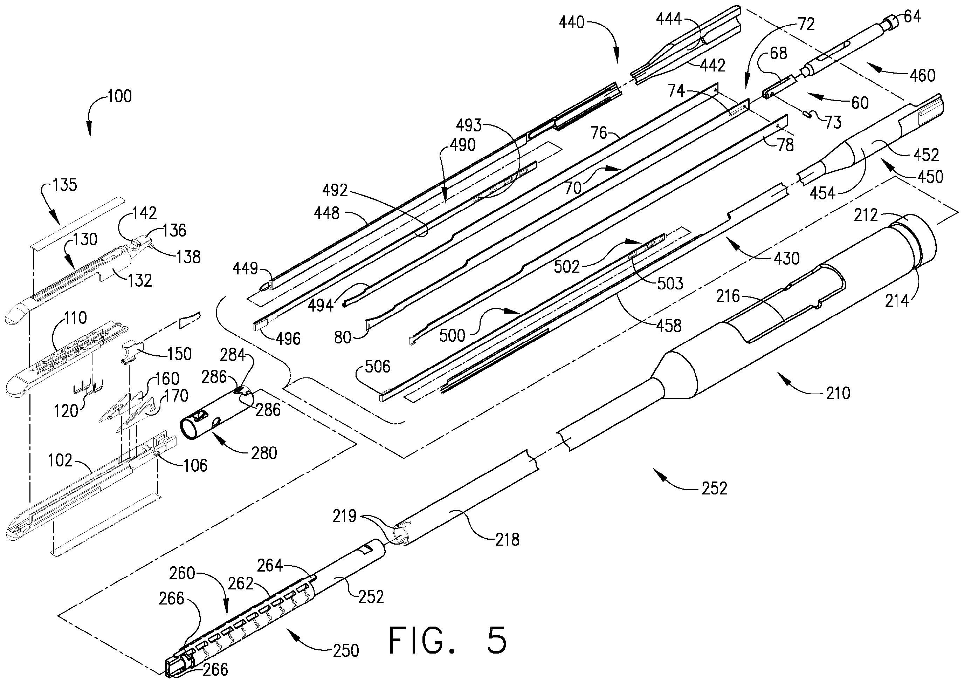

The closure drive system 200 further includes an intermediate flexible tube segment 250 that is configured for attachment to the distal end 218 of the proximal closure tube segment 210. As can be seen in FIG. 5, the intermediate tube segment 250 includes a flexible articulation portion 260 and an attachment stem portion 252. The attachment stem portion 252 is sized to extend into the open distal end 218 of the proximal closure tube segment 210 in frictional engagement therewith. The flexible articulation portion 260 is integrally formed with the attachment stem portion 252 and includes an articulation spine 262 that includes proximal end portions 264 (only one is shown in FIG. 5) that are configured to be received in corresponding notches 219 in the distal end 218 of the proximal closure tube segment 210 to prevent relative rotation between the proximal closure tube segment 210 and the intermediate tube segment 250. The intermediate tube segment 250 is non-rotatably (i.e., attached to prevent relative rotation between these components) attached to the proximal closure tube segment 210 by, for example, screws, detents, adhesive, etc.

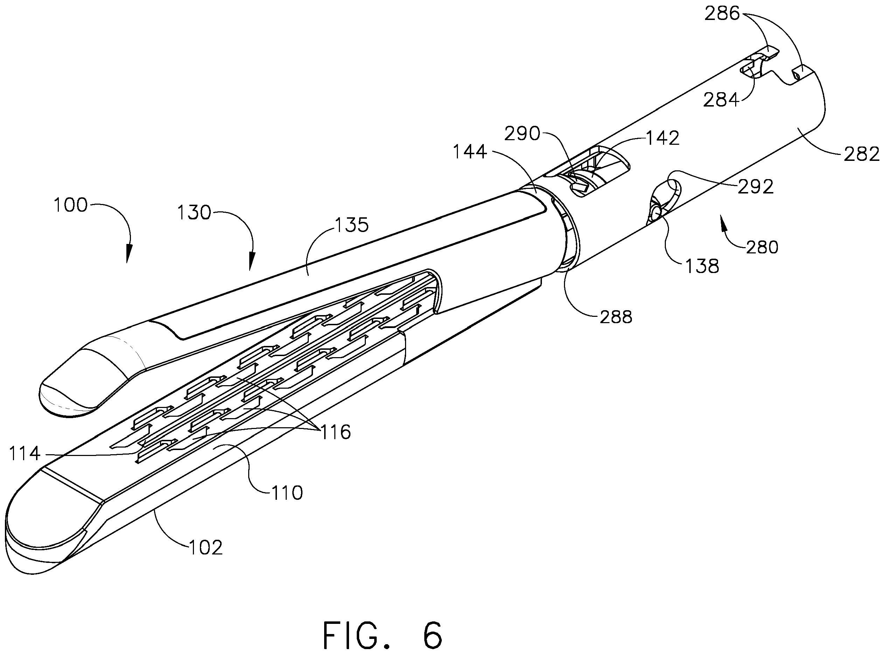

The closure drive system 200 further include a distal closure tube segment 280 that is configured to axially engage and apply opening and closing motions to the anvil assembly 130. The distal closure tube segment 280 is attached to the distal end of intermediate tube segment 250 for axial travel therewith. The articulation spine 262 further include distal end portions 266 that are configured to be received in corresponding notches 284 in the proximal end 282 of the distal closure tube segment 280 to prevent relative rotation between the distal closure tube segment 280 and the intermediate tube segment 250. See FIG. 5. The proximal end 282 of the distal closure tube segment 280has inwardly extending attachment tabs 286 that are adapted to be bent into corresponding notches 266 in the intermediate tube segment 250. See FIGS. 5 and 6. Such arrangement serves to facilitate attachment of the distal closure tube segment 280 to the intermediate tube segment 250 for axial travel therewith.

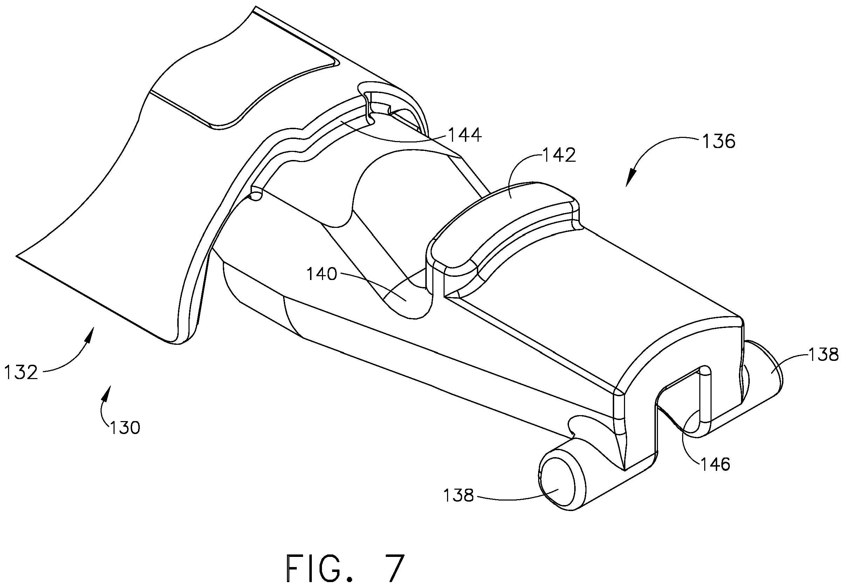

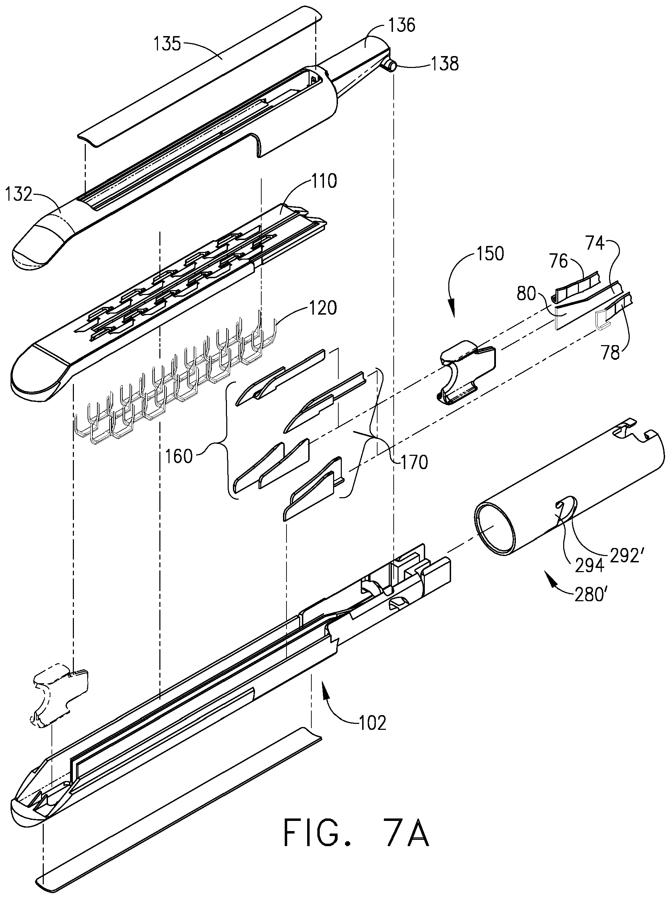

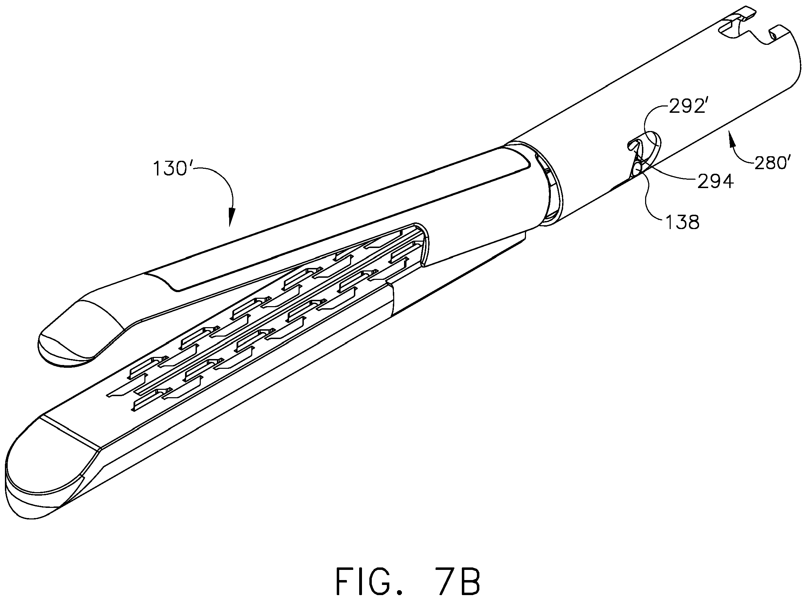

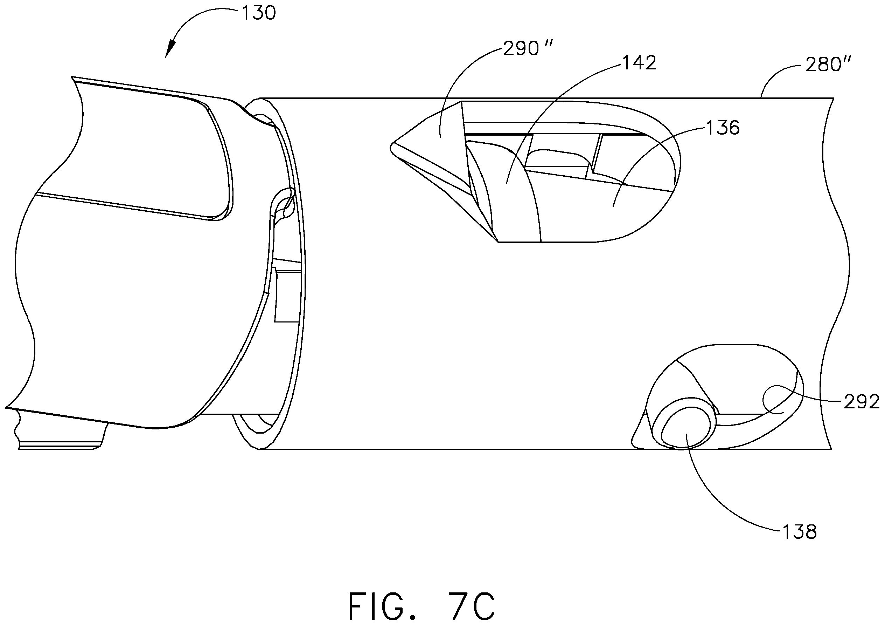

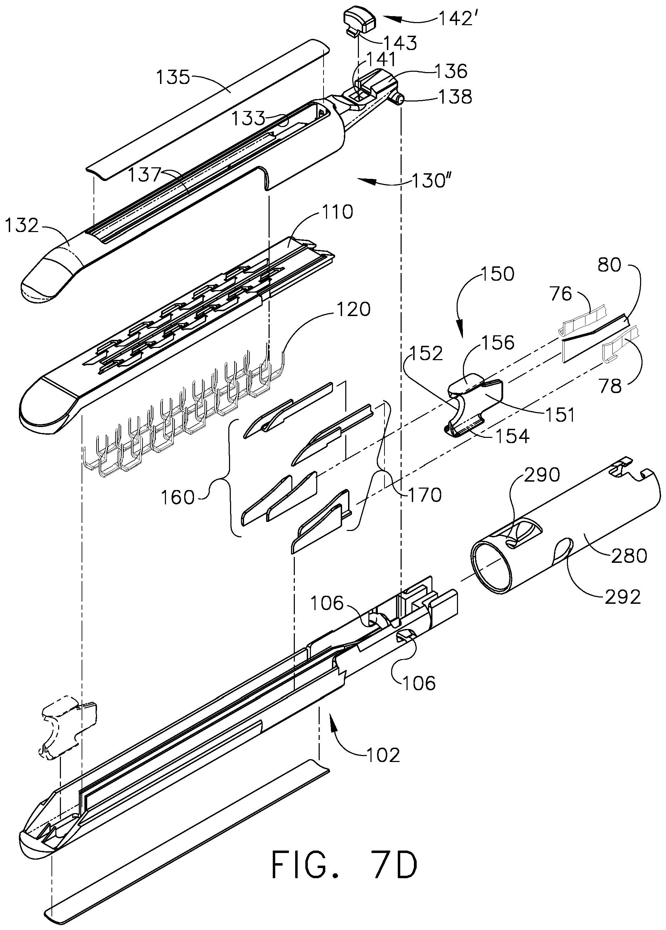

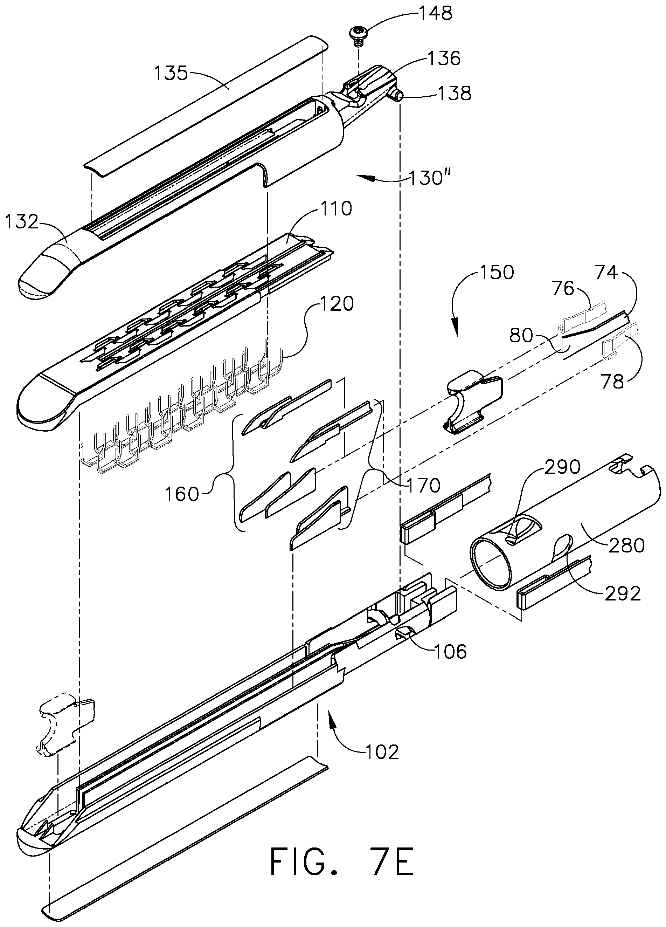

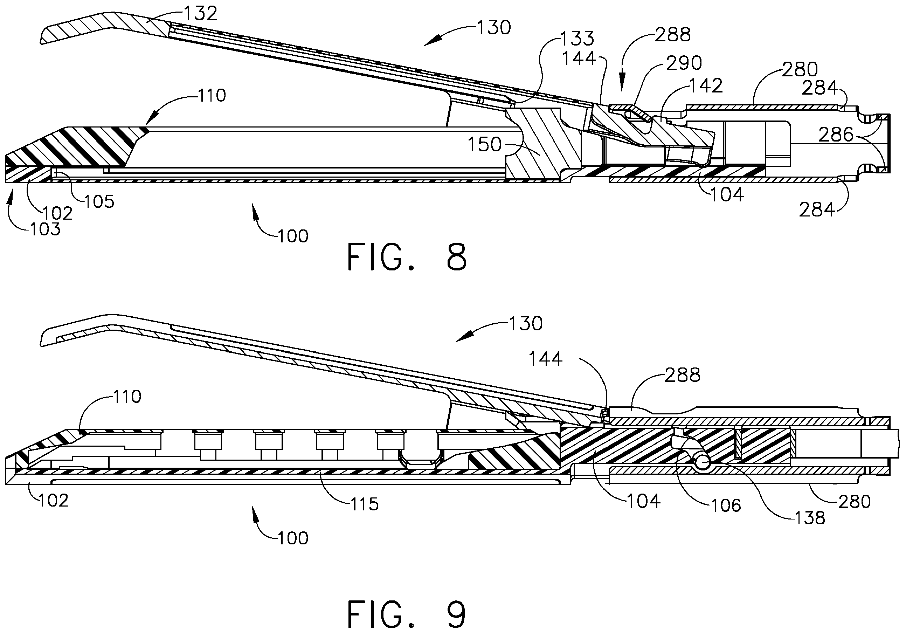

The distal closure tube segment 280 is configured to apply opening and closing motions to the anvil assembly 130. As can be seen in FIG. 7, one form of the anvil mounting portion 136 is formed with a groove 140 that defines an anvil tab 142. As can be seen in FIGS. 6 and 8, the distal end 288 of the distal closure tube segment 280 has an inwardly extending actuation tab 290 formed therein that is configured to interact with the anvil tab 142. For example, when the distal closure tube segment 280 is in the open position (FIGS. 6 and 8), the actuation tab 290 is in biasing contact with the anvil tab 142 which serves to pivot the anvil assembly 130 to the open position. As shown in FIG. 9, when the anvil assembly 130 is in an open position, the trunnions 138 are located in the bottom of the trunnion slots 106 in the proximal mounting portion 104 of the elongated channel 102. When the distal closure tube segment 280 is advanced distally, the distal end 288 contacts an upstanding end wall 144 on the anvil body 132 to cause the anvil assembly 130 to pivot or otherwise move toward the surgical fastener cartridge 110. When assembled, the trunnions 138 each extend into a corresponding opening 292 in the distal closure tube segment 280. See FIG. 6.

Operation of the closure drive system 200 will now be described. The anvil assembly 130 is moved relative to the surgical fastener cartridge 110 by pivoting the closure trigger toward and away from the pistol grip portion 28 of the handle 22. Thus, actuating the closure trigger 202 causes the proximal closure tube segment 210, the intermediate tube segment 250 and the distal closure tube segment 280 to move axially in the distal direction "DD" to contact the end wall 144 of the anvil body portion 132 to pivot or otherwise move the anvil 130 toward the surgical fastener cartridge 110. The clinician grasp and manipulate tissue between the anvil assembly 130 and the fastener cartridge 110 by opening and closing the anvil assembly 130. Once the target tissue is captured between the anvil assembly 130 and fastener cartridge 110, the clinician pivot the closure trigger 202 to the fully actuated position wherein it is locked in place for firing.

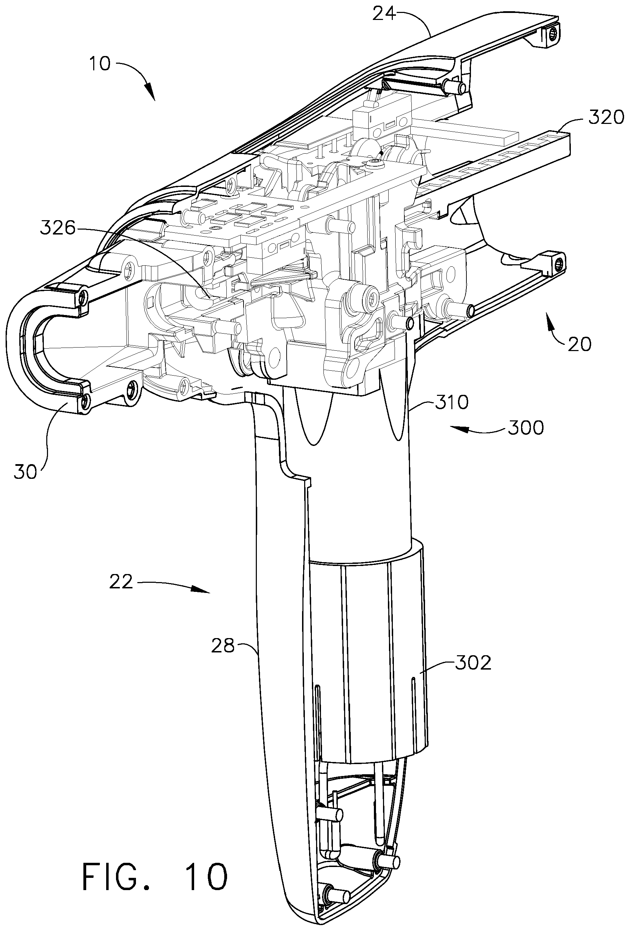

As indicated above, the frame 30 also is configured to operably support the firing drive system 300 that is configured to apply firing motions to corresponding portions of the elongated shaft assembly 50 and ultimately to the knife assembly 150 and the sled assemblies 160, 170. As can be seen in FIGS. 3 and 10, the firing drive system 300 employs an electric motor 302 that is supported in the pistol grip portion 28 of the handle 22. The motor 302 is a DC brushed driving motor having a maximum rotation of, approximately, 25,000 RPM, for example. In other arrangements, the motor 302 include a brushless motor, a cordless motor, a synchronous motor, a stepper motor, or any other suitable electric motor. A battery 304 (or "power source" or "power pack"), such as a Li ion battery, for example, is coupled to the handle 22 to supply power to a control circuit board assembly 306 and ultimately to the motor 302. FIG. 3 illustrates a battery pack housing 305 that is configured to be releasably mounted to the handle 22 for supplying control power to the surgical instrument 10. A number of battery cells connected in series are used as the power source to power the motor 302. In addition, the power source is replaceable and/or rechargeable.

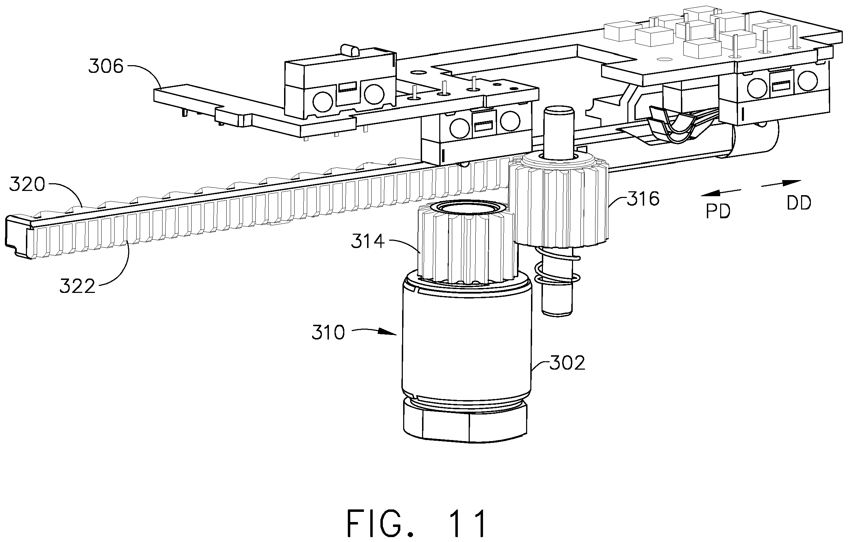

As outlined above with respect to other various forms, The electric motor 302 includes a rotatable shaft 308 that operably interfaces with a gear reducer assembly 310 that is mounted in meshing engagement with a set, or rack, of drive teeth 322 on a longitudinally-movable drive member 320. The gear reducer assembly 310 include, among other things, a housing 312 and an output pinion gear 314. See FIG. 11. The output pinion gear 314 is directly operably engaged with the longitudinally-movable drive member 320 or, alternatively, operably engaged with the drive member 320 via one or more intermediate gears 316. The intermediate gear 316 is meshingly engaged with the set, or rack, of drive teeth 322 defined in the drive member 320. In use, the electric motor 302 move the drive member distally, indicated by an arrow "DD", and/or proximally, indicated by an arrow "PD", depending on the direction in which the electric motor 302 rotates the intermediate gear 316. In use, a voltage polarity provided by the battery operate the electric motor 302 in a clockwise direction wherein the voltage polarity applied to the electric motor by the battery is reversed in order to operate the electric motor 302 in a counter-clockwise direction. When the electric motor 302 is rotated in one direction, the drive member 320 will be axially driven in the distal direction "DD". When the motor 302 is driven in the opposite rotary direction, the drive member 320 will be axially driven in a proximal direction "PD". The handle 22 includes a switch which is configured to reverse the polarity applied to the electric motor 302 by the battery. The handle 22 also includes a sensor that is configured to detect the position of the movable drive member 320 and/or the direction in which the movable drive member 320 is being moved.

Actuation of the motor 302 is controlled by a firing trigger 330 that is pivotally supported on the handle 22. The firing trigger 330 is pivoted between an unactuated position and an actuated position. The firing trigger 330 is biased into the unactuated position by a spring (not shown) or other biasing arrangement such that when the clinician releases the firing trigger 330, it is pivoted or otherwise returned to the unactuated position by the spring or biasing arrangement. The firing trigger 330 is positioned "outboard" of the closure trigger 202 as discussed in further detail in U.S. Patent Application Publication No. 2014/0263542 A1. In at least one form, a firing trigger safety button 332 is pivotally mounted to the closure trigger 202. The safety button 332 is positioned between the firing trigger 330 and the closure trigger 202 and has a pivot arm (not shown) protruding therefrom. When the closure trigger 202 is in the unactuated position, the safety button 332 is contained in the handle housing where the clinician cannot readily access it and move it between a safety position preventing actuation of the firing trigger 330 and a firing position wherein the firing trigger 330 is fired. As the clinician depresses the closure trigger 202, the safety button 332 and the firing trigger 330 pivot down to a position wherein they then are manipulated by the clinician.

As indicated above, the longitudinally movable drive member 320 has a rack of teeth 322 formed thereon for meshing engagement with a corresponding drive gear 312 of the gear reducer assembly 310. A manually-actuatable "bailout" assembly is configured to enable the clinician to manually retract the longitudinally movable drive member 320 should the motor become disabled. U.S. Patent Application Publication No. 2014/0263542 A1 contains further details of one form of bailout assembly that is employed. U.S. Patent Application Publication No. US 2010/0089970 also discloses "bailout" arrangements and other components, arrangements and systems that may also be employed with the various instruments disclosed herein. U.S. Patent Application Publication No. 2010/0089970, entitled POWERED SURGICAL CUTTING AND STAPLING APPARATUS WITH MANUALLY RETRACTABLE FIRING SYSTEM, now U.S. Patent Application, is also relevant.

Referring to FIGS. 4 and 5, the elongated shaft assembly 50 includes a firing member assembly 60 that is supported for axial travel within an articulation shaft assembly 430 that is part of the articulation system 400 and which essentially functions as shaft frame or spine. The firing member assembly 60 further include a proximal firing shaft 62 that has a proximal end portion 64 that is configured to be rotatably received in a distal cradle 326 provided in a distal end 324 of the movable drive member 320. Such arrangement permits the proximal firing shaft 62 to rotate relative to the movable drive member 320 while also axially moving therewith. The proximal firing shaft 62 further has a slot 68 formed in its distal end 66 for receiving a proximal end 72 of a flexible distal firing shaft assembly 70 therein. See FIG. 5. As can be seen in that Figure, the proximal end 72 of the distal firing shaft assembly 70 is received within the slot 68 in the distal firing shaft 62 and is pinned thereto with a pin 73.

The distal firing shaft assembly 70 includes a central firing beam 74 that is located between a right sled pusher beam 76 and a left sled pusher beam 78. The central firing beam 74 and the pusher beams 76, 78, for example, each is fabricated from metal that facilitates axial actuation of the sled assemblies 160, 170 in the surgical end effector 100 while also facilitating flexing thereof when the end effector 100 is articulated as will be discussed in further detail below. In at least one arrangement, the central pusher beam 74, the right sled pusher beam 76 and the left sled pusher beam 78 extend through a slot 146 in the anvil mounting portion 136. The right sled pusher beam 76 corresponds to the right sled assembly 160 and the left sled pusher beam 78 corresponds to the left sled assembly 170 movably supported within the elongated channel 102. Axial movement of the right sled pusher beam 76 and the left sled pusher beam 78 will result in the axial advancement of the right and left sled assemblies 160, 170, respectively, within the elongated channel 102. As the right sled assembly 160 is axially advanced within the elongated channel 102, it drives the surgical fasteners 120 supported in the cartridge body 112 on the right side of the slot 114 out of their respective pockets 116 and as the left sled assembly 170 is axially advanced within the elongated channel 102, it drives the surgical fasteners 120 supported within the cartridge body 112 on the left side of the slot 114 out of their respective pockets 116.

The central firing beam 74 has a distal end 80 that is configured to be received within a slot 151 provided in the knife assembly 154 and retained therein by, for example, a frictional fit, adhesive, welding, etc. A bottom window 105 is formed in a distal end 103 of the elongated channel 102 to enable the knife assembly 150 to be inserted therethrough. The elongated channel 102 is formed with a right upstanding wall 107 and a left upstanding wall 108 that define a centrally-disposed channel slot 109. Once the knife assembly 150 is inserted into the bottom window 105 in the elongated channel 102, the body portion 151 of the knife assembly 150 is inserted into the channel slot 109 and advanced proximally in the elongated channel 102 to be coupled with the distal end 180 of the central firing beam 74. A lower channel cover 111 is attached to the bottom of the elongated channel 102 to prevent tissue, body fluids, etc. from entering into the elongated channel 102 which might hamper the movement of the knife assembly 150 therein.

The anvil assembly 130 is installed onto the elongate channel 102 as follows. To commence the installation process, the anvil assembly 130 is positioned over the elongated channel 102 such that the trunnions 138 can be inserted into notches 113 in the proximal mounting portion 104 of the elongated channel 102 which enable the trunnions 138 to enter the corresponding trunnion slots 106 in the elongated channel 102. See FIG. 2. This installation is performed before the distal closure tube segment 280 has been attached to the intermediate tube segment 250 or after the distal closure tube segment 280 has been moved sufficiently proximally to permit the anvil to be so positioned. Once the trunnions 138 are received within their respective trunnion slots 106, the distal closure tube segment 280 is moved to the position shown in FIGS. 8 and 9 wherein the distal closure tube segment 280 retains the trunnions 138 in their respective trunnion slots 106 and the actuation tab 290 is in biasing contact with the anvil tab 142 which serves to pivot the anvil assembly 130 to the open position. When in that position, each trunnion 138 protrudes into a corresponding opening 192 in the distal closure tube segment 280. See FIG. 6. As shown in FIGS. 2 and 8, when the anvil assembly 130 is in an open position, the upper end of the knife assembly 150 enters a window 133 in the anvil body portion 132. Such window 133 provides clearance for the anvil assembly 130 to be moved to the closed positions while the knife assembly 150 remains in the unactuated position. Once the anvil assembly 130 has been installed with the knife assembly 150 in place, an anvil cover 135 is attached to the anvil body 134 to prevent tissue, body fluids, etc. from entering into the anvil body 134 which might hamper the movement of the knife assembly 150 therein. As the knife assembly 150 is advanced distally in the end effector 100, the upper tab 156 of the knife assembly 150 engages ledges in the anvil body and the lower foot 154 engages portions 115 of the elongated channel 102 and serves to retain the anvil assembly 130 in the closed position and essentially maintain the spacing between the anvil assembly 130 and the fastener cartridge 110.

FIGS. 7A and 7B illustrate an alternative distal closure tube arrangement 280' that work with an anvil assembly 130' that is substantially identical to anvil assembly 130 except that anvil assembly 130' lacks an anvil tab. In such an arrangement, for example, each trunnion 138 extends into a corresponding opening 292' in the distal closure tube segment 280'. The distal closure tube segment 280 further includes an inwardly extending gill tab 294 that protrudes inward for contact with the corresponding anvil trunnion 138. When the distal closure tube segment 280' is drawn in the proximal direction "PD", each gill tab 294 contacts the corresponding trunnion 138 to cause the trunnion to move downwardly in its corresponding trunnion slot 106 in the elongated channel 102 to pivot or otherwise move the anvil assembly 130' into open positions. FIG. 7C illustrates yet another distal closure tube arrangement 280" wherein the actuation tab is formed by an indentation 290" in the distal closure tube segment 280" for interaction with the anvil tab 142 in the above-described manner.

FIG. 7D illustrates an alternative anvil assembly 130" wherein the anvil tab 142' is removably attached to the anvil mounting portion 136. In one arrangement for example, the anvil tab 142' is configured with a snap tab 143 arranged to retainingly engage an opening 141 in the anvil mounting portion 136. The anvil assembly 130" otherwise are the same as anvil assembly 130 described above and is opened and closed in similar manners by the distal closure tube segment 280. FIG. 7E illustrates yet another anvil assembly 130" wherein the anvil tab is formed by a screw 148 that is removably attachable to the anvil mounting portion 138. Such removable anvil tab/screw arrangements facilitate ease of installation of the anvil assembly 130'.

Referring to FIGS. 4 and 5, one form of articulation system 400 includes an articulation shaft assembly 430 that is operably controlled by an articulation control system 460. For example, the articulation shaft assembly 430 includes a right articulation shaft segment 440 and a left articulation shaft segment 450. The right articulation shaft segment 440 includes a proximal end 442 that has a right passage segment 444 formed therein. Likewise the left articulation shaft segment 450 includes a proximal end portion 452 that has a left passage segment 454 formed therein. When the right articulation shaft segment 440 and the left articulation shaft segment 450 are installed within the proximal closure tube segment 210, they form the articulation shaft assembly 430. The right passage segment 444 and the left passage segment 454 cooperate to receive a portion of the proximal firing shaft 62 therein. The right articulation shaft segment 440 and the left articulation shaft segment 450 are, for example, composed of a plastic, especially a glass fibre-reinforced amorphous polyamide, sold commercially under the trade name Grivory GV-6H by EMS-American Grilon.

In various arrangements, for example, the articulation control system 460 includes a nozzle assembly 462 that is supported for rotational travel relative to the handle 22. As can be seen in FIG. 4, the nozzle assembly 462 comprises an upper nozzle segment 464 and a lower nozzle segment 466 that are attached together by a series of fasteners (e.g., screws) 468. The upper nozzle segment 464 is configured to rotatably support an articulation control knob 470 thereon. In one arrangement, for example, the articulation control knob 470 extends through an opening (not shown) in the upper nozzle segment 464 and is coupled to an articulation gear member 472 by screws 474. The articulation gear member 472 includes articulation spur gear 476 that extends into an opening 216 in the proximal end portion 212 of the proximal closure tube segment 210. As may further be seen in FIG. 4, the articulation system 400 further includes a right actuation tube adapter 478 and a left articulation tube adapter 480. The right articulation tube adapter 478 has a right recess 479 formed therein that is adapted to receive a right adapter lug 446 formed on the proximal end 442 of the right articulation shaft segment 440. Likewise, the left articulation tube adapter 480 includes a left recess 482 that is adapted to engage a left adapter lug 456 formed on the proximal end 452 of the left articulation shaft segment 450. The right articulation tube adapter 478 further has a series of right articulation drive gears 481 that are configured for meshing engagement with the articulation spur gear 476. The left articulation tube adapter 480 has a series of left articulation drive gears 484 formed therein that are adapted to intermesh with the articulation spur gear 476. Thus, when the articulation control knob 470 is rotated about a control axis CA-CA that is transverse to the longitudinal tool axis LT-LT relative to the handle 22 (FIG. 1), the left articulation shaft segment 450 is, for example, driven axially in the distal direction "DD" within the proximal closure tube segment 210 and the right articulation shaft segment 440 is simultaneously axially driven in the proximal direction "PD".

Still referring to FIG. 5, the articulation shaft assembly 430 further include a right articulation band 490 and a left articulation band 500. A proximal end portion 492 of the right articulation band 490 is attached to a distal portion 448 of the right articulation shaft segment such that a distal portion 494 of the right articulation band 490 protrudes out of a right passage 449 therein. The proximal end portion 492 of the right articulation band 490 includes holes or cavities 493 that are configured to receive corresponding lugs (not shown) in the right articulation shaft segment 440 to facilitate attachment of the right articulation band 490 to the right articulation shaft segment 440. Likewise, a proximal end portion 502 of the left articulation band 500 has holes or cavities 503 therein that are configured to receive lugs (not shown) in the distal portion 458 of the left articulation shaft segment 450 to facilitate attachment of the left articulation band 500 to the articulation shaft segment 450. The articulation bands 490 and 500 are composed of a metal, advantageously full hard 301 stainless steel or its equivalent.

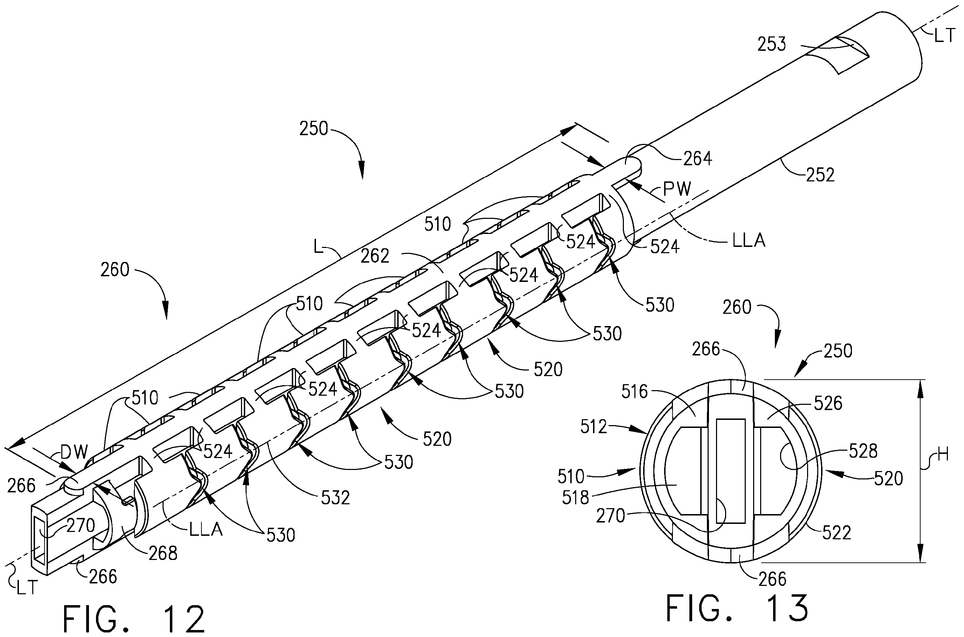

Referring now to FIGS. 12-15, as was briefly discussed above, the intermediate tube segment 250 has an attachment stem portion 252 and a flexible articulation portion 260. In various arrangements, the intermediate tube segment 250 is fabricated from, for example, rigid thermoplastic polyurethane sold commercially as ISOPLAST grade 2510 by the Dow Chemical Company and include a centrally disposed, vertically-extending articulation spine 262. The articulation spine 262 includes a proximal spine end 264 and a distal spine end 266 that facilitate attachment to the proximal closure tube segment 210 and the distal closure tube segment 280, respectively as was discussed above. The articulation spine 262 further includes a centrally disposed component or knife slot 270 for facilitating the passage of various control components therethrough. In the illustrated arrangement, the slot 270 movably supports the central firing beam 74, the right pusher beam 76 and the left pusher beam 78. The centrally disposed slot 270 is substantially enclosed to retard or prevent infiltration of body fluids and tissue therein which might otherwise hamper the movement of the control components operably passing therethrough.

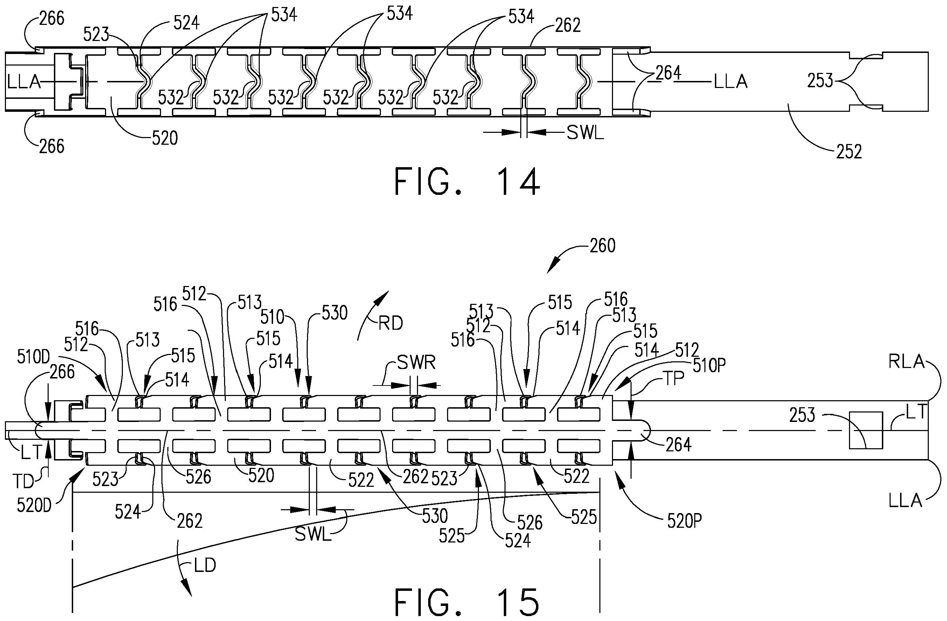

As can most particularly be seen in FIG. 15, the flexible articulation portion 260 further includes a plurality of right ribs 510 and a plurality of left ribs 520 that are integrally-formed with, and laterally protrude from, the articulation spine 262. In various forms, for example, each right rib 510 comprises a rib body portion 512 that is spaced from the articulation spine 262 by a corresponding right rib neck portion 516. Likewise, each left rib 520 comprises a left rib body portion 522 that is spaced from the articulation spine 262 by a left rib neck portion 526. As can be seen in FIG. 13, the right and left rib body portions 512, 522 have an arcuate shape to provide the flexible articulation portion 260 of the intermediate tube segment 250 with a substantially-circular cross-sectional shape. Such shape facilitates easy passage of the intermediate tube segment 250 through a circular passage such as, for example, an appropriately sized trocar.

In various arrangements, each of the right rib neck portions 516 serves to define a right articulation passage 518 for movably receiving the right articulation band 490 therethrough. The right articulation band 490 extends through the right articulation passage 518 and is coupled to the proximal mounting portion 104 of the elongate channel 102. For example, the distal end 494 of the right articulation band 490 has a right hook portion 496 that is adapted to be coupled to a right attachment portion 497 of the elongated channel 102. See FIG. 2. Similarly, each of the left rib neck portions 526 serves to define a left articulation passage 528 for movably receiving the left articulation band 500 therethrough. The left articulation band 500 extends through the left articulation passage 528 and is coupled to the proximal mounting portion 104 of the elongated channel 102. For example, the distal end 504 of the left articulation band 500 has a left hook portion 506 that is adapted to be coupled to a left attachment portion 507 of the elongated channel 102.

One method of operating the articulation system 400 will now be described. When the clinician wishes to articulate the end effector 100 to the right relative to the longitudinal tool axis LT-LT (the right direction is represented by arrow "RD" in FIG. 15), the clinician simply rotates the articulation control knob 470 in the appropriate direction. For example, turning the control knob 470 in a clockwise direction (when viewed from above) causes the left articulation band to be pushed in the distal direction "DD" and the right articulation band 490 is drawn in the proximal direction "PD" which serve to apply an articulation motion to the elongated channel 102. As the articulation motion is applied to the elongated channel 102, the flexible articulation portion 260 flexes to accommodate the movement of the surgical end effector 100 in the "right" direction. Conversely, if the clinician wishes to articulate the end effector 100 in the left direction "LD", the clinician simply rotates the control knob 470 in a counterclockwise direction which causes the right articulation band 490 to be pushed in the distal direction "DD" and the left articulation band 500 to be drawn in the proximal "PD" direction thereby causing the surgical end effector 100 to move to the left. The end effector 100 may also be articulated by a robotic system (not shown) that is configured to apply control motions to the articulation bands 490, 500.

Upon application of the above-described articulation motions to the surgical end effector 100, it is desirable to avoid twisting or torquing the flexible articulation portion 260 of the intermediate tube segment 250. If such torque or twisting were to occur, the possibility exists for hampering or, in instances of severe twisting, completely jamming the operation of the central firing beam 74 and the right and left sled pusher beams 76, 78. To avoid this problem, the right and left ribs 510, 520 are uniquely configured to prevent twisting between the ribs.

In at least one arrangement, for example, each rib body 512 has lateral ends that are arranged in spaced, confronting relationship with the lateral ends of the rib bodies of adjacent ribs. Referring again to FIG. 15, for example, the rib body 512 of each right rib 510 has a first right lateral end 513 and a second right lateral end 514. With the exception of the proximal-most right rib 510P and the distal-most right rib 510D, the first right lateral end 513 of one right rib 510 is in confronting relationship with the second right lateral end 514 of an adjacent right rib 510. When the flexible articulation portion 260 of the intermediate tube segment 250 is unarticulated (e.g., the flexible articulation portion 260 is substantially axially aligned on the longitudinal tool axis LT-LT), the first right lateral end 513 of each right ribs 510 is spaced from the second right lateral end 514 of the adjacent right rib 510 by a right rib space 515. In the arrangement depicted in FIG. 15, for example, all of the right rib spaces 515 have substantially the same space width "SWR". Likewise, the rib body 522 of each left rib 520 has a first left lateral end 523 and a second left lateral end 524. With the exception of the proximal-most left rib 520P and the distal most left rib 520D, the first left lateral end 523 of one left rib 520 is in confronting relationship with the second left lateral end 524 of an adjacent left rib 520. When the flexible articulation portion 260 of the intermediate tube segment 250 is unarticulated, the first left lateral end 523 of each left rib 520 is spaced from the second left lateral end 524 of the adjacent left rib 520 by a left rib space 525. In the arrangement depicted in FIG. 15, for example, all of the left rib spaceS 525 HAVE substantially the same space width "SWL". The right rib space widths SWR are substantially the same as the left rib space widths SWL. However, the right and left rib space widths may differ from each other.

Still referring to FIG. 15, each rib is provided with a twist-preventing configuration, generally designated as 530. In at least one arrangement, for example, an anti-twist protrusion 532 is formed on each of the first right lateral ends 513 of the right rib bodies 512 and on each of the first left lateral ends 523 of each of the left rib bodies 522. Each anti-twist protrusion 532 corresponds with a substantially complementary-shaped recess 534 formed in the rib that is immediately adjacent and in confronting relationship therewith. FIG. 14 illustrates this arrangement on the left ribs 520. In at least one arrangement, the right ribs 510 employ an identical configuration. The protrusions 532 are substantially aligned along a lateral axis. That is, the protrusions 532 formed on the right ribs 510 are substantially aligned along a right lateral axis RLA-RLA on the right side of the articulation spine 262 and the protrusions 532 formed on the left ribs 520 are substantially aligned on the left side of the articulation spine 262 along a left lateral axis LLA-LLA. When the flexible portion 260 is unarticulated, the right lateral axis RLA-RLA, the left lateral axis LLA-LLA and the longitudinal tool axis LT-LT are substantially parallel to each other. As we see in FIG. 15, the right lateral axis RLA-RLA and the left lateral axis LLA-LLA are spaced from the longitudinal tool axis LT-LT.

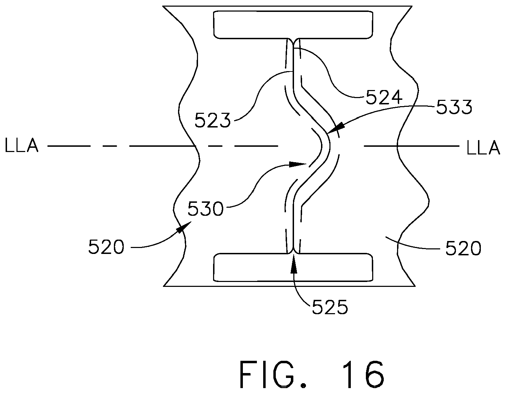

As the flexible articulation portion 260 is articulated in the right direction "RD", at least some of the protrusions 532 on the right ribs 510 will frictionally engage a portion of a corresponding recess 532 in an adjacent right rib 510 to prevent the flexible portion 260 from twisting. Similarly, as the flexible articulation portion 260 is articulated in the left direction "LD", at least some of the protrusions 532 on the left ribs 520 will engage a portion of the recess 532 in an adjacent left rib 520 in a "twist-preventing orientation" to prevent the flexible portion 260 from twisting. This engagement/orientation between the protrusion 532 and the bottom of the cavity 534 in an adjacent left rib 520, for example, is illustrated in FIG. 16. As can be seen in that Figure, in that example, the first left lateral end 523 of one of the second rib 520 is in abutting contact with the second left lateral end 524 of an adjacent left rib 520 to thereby prevent or retard twisting of the flexible portion 260 of the intermediate tube segment 250.

Various alternative anti-twist arrangements are also contemplated. For example, the anti-twist features not provided on, for example, the proximal-most four ribs. In still other arrangements, the anti-twist features are provided in a plurality of ribs comprising a central area of the flexible segment, but not in the proximal-most and distal most ribs. In, other arrangements, the ant-twist features are employed on every other pair of ribs along the length of the flexible segment. For example, the proximal-most pair of adjacent ribs have anti-twist features, then the next rib or ribs (distal to those ribs) not have anti-twist features and the next ribs (distal thereto) have the anti-twist features and so on. These alternative arrangements may be applied only to the ribs on one side of the articulation spine or they may be employed on the ribs on both sides of the articulation spine. By altering the number, location and/or spacing of the ribs with the anti-twist features, as well as the space widths between the ribs (with and without anti-twist features), as well as the geometric shape of the articulation spine, one advantageously adjust the overall flexibility of the flexible segment, its degree of articulation, its degree of stiffness and its rate of articulation.

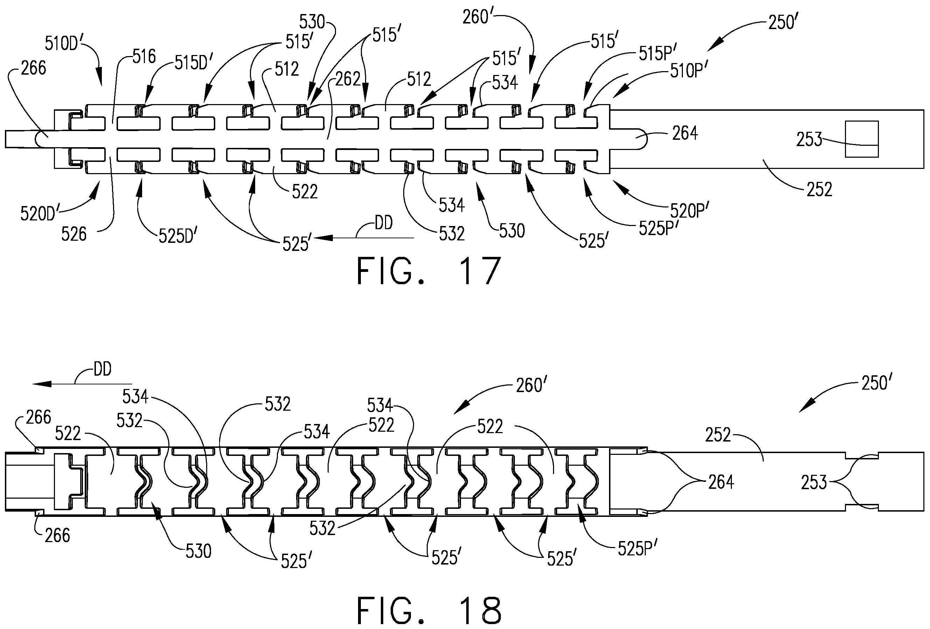

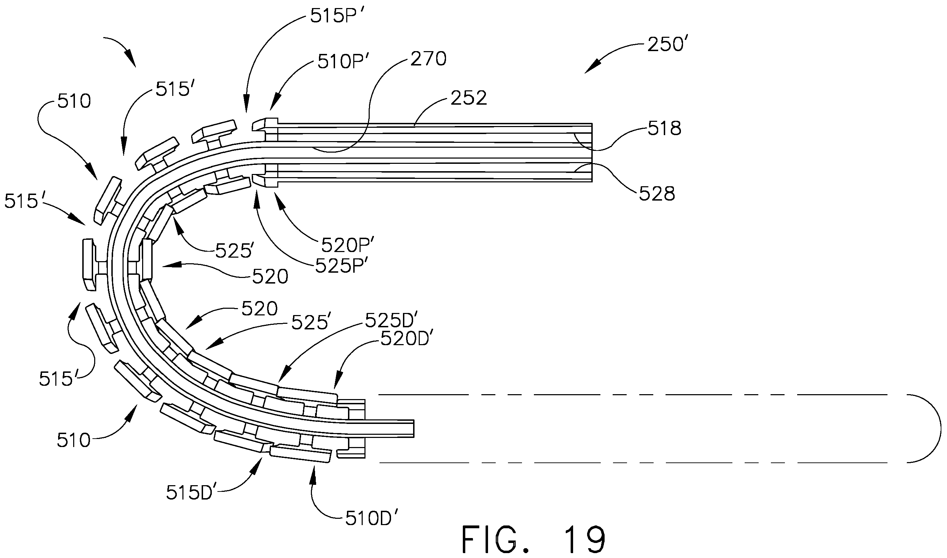

Referring to FIGS. 12 and 13, in the illustrated arrangement, the articulation spine 262 is elongated and has a height, generally designated as "H". In at least one arrangement, the height "H" is substantially consistent for the length "L" of the articulation spine 262. In addition, the articulation spine 262 decreasingly tapers from the proximal end portion 264 to the distal end portion 266. More specifically, as can be seen in FIG. 12, the proximal end portion 262 has a proximal width "PW" and the distal end portion 266 has a distal width "DW". The "PW" is greater than the distal width "DW" and the width of the articulation spine gradually tapers in width (as opposed to height) from the proximal end 264 to the distal end 266 along length "L". Such tapered articulation spine arrangement further serves to retard twisting during articulation of the surgical end effector while facilitating increased articulation of the distal end of the flexible portion 260 relative to the proximal end of the flexible portion 260 and while facilitating movable passage of various control components (e.g., central firing beam 74, right sled pusher beam 76, left sled pusher beam78, etc.) therethrough.
Further, in one arrangement, when the flexible portion 260 is in an unarticulated or flexed position, all of the right rib spaces 515 and left rib spaces 525 have the same starting width. Thus, in that configuration, SWR=SWL. FIGS. 17 and 18 illustrate an intermediate tube segment 250' according to the present invention that is substantially identical to the intermediate tube segment 250' described above, except that the right rib spaces 515' and the left rib spaces 525' decrease in magnitude going from the proximal end of the flexible articulation portion 260' to the distal end of the flexible articulation portion 260. That is, the proximal-most right rib space 515P' is the widest right rib space and the distal most right rib space 515D' is the narrowest right rib space with the right rib spaces 515' getting successively narrower going in the distal direction "DD". Similarly, the proximal-most left rib space 525P' is the widest left rib space and the distal-most left rib space 525D' is the narrowest left rib space with the left rib spaces 525' getting successively narrower going in the distal direction. In such arrangement, when the articulation motion is applied to the surgical end effector, the flexible portion 260' will have a faster rate of flexure at its distal end. That is, a distal portion of flexible segment 260' will flex or articulate at a rate that is greater than a rate at which another portion of 260' that is proximal to that distal segment will articulate upon application of an articulation motion to the end effector. Relative movement between the ribs on the distal end will stop before the relative movement between the more proximal ribs stops because the spaces between the distal ribs are smaller than the spaces between the proximal ribs. In the illustrated arrangement the widths of the right and left rib spaces 515' and 525' that are laterally aligned with each other are equal in magnitude. Such rib space width arrangements enable the flexible articulation portion 260' to assume a substantial "U"- shape if desired. See e.g., FIG. 19. It will be understood, however, that various other slot width arrangements, sizes and configurations are employed to achieve a desired amount/range of articulation while preventing the intermediate tube from inadvertently twisting about the longitudinal tool axis.
The invention is used in connection with a variety of different forms of surgical end effector to effectuate articulation and operation of such end effectors without incurring twist that might hamper the operation of control components interfacing between the end effector and the various control systems. For example, there is provided a surgical instrument that includes a surgical end effector and an elongated shaft assembly that defines a longitudinal tool axis. The elongated shaft assembly is operably coupled to the surgical end effector and includes a flexible segment that has a proximal end and a distal end. The flexible segment is configured such that a distal portion thereof articulates at an articulation rate that is fastener than another articulation rate of another portion of the flexible segment that is proximal to the distal portion upon application of articulation motions to the surgical end effector.

In accordance with another general form, there is provided a surgical instrument that includes a surgical end effector that is configured to perform at least one surgical procedure upon application of at least one control motion thereto from at least one control component. An elongated shaft assembly define a longitudinal tool axis and is operably coupled to the surgical end effector. The elongated shaft assembly operably support at least one of the control components and include a flexible articulation portion for facilitating articulation of the surgical end effector relative to the longitudinal tool axis upon application of articulation motions to the surgical end effector. The flexible articulation portion comprises a longitudinally-extending, elongated articulation spine that includes two lateral sides. A plurality of ribs extends from each lateral side of the articulation spine along a length thereof. Each of the rib include at least one anti-twist feature formed therein for preventing the flexible articulation portion from twisting about the longitudinal tool axis during articulation of the surgical end effector relative to the tool axis.

In accordance with at least one other general form, there is provided a surgical instrument that include a housing that operably supports at least one control system that is configured to selectively generate at least one control motion. The surgical instrument further comprises a surgical end effector that is configured to perform at least one surgical procedure upon application of at least one control motion thereto. An elongated shaft assembly are operably coupled to the housing and surgical end effector and define a longitudinal tool axis. The elongated shaft assembly include a flexible articulation portion that comprises a longitudinally-extending, elongated articulation spine that includes two lateral sides. A plurality of ribs extends from each lateral side of the articulation spine along a length thereof. Each rib include at least one anti-twist feature for preventing the flexible articulation portion from twisting about the longitudinal tool axis during articulation of the surgical end effector relative to the tool axis.

In accordance with at least one other general form, there is provided a surgical instrument that include an end effector that comprises an elongated channel that is configured to support a surgical fastener cartridge that includes a plurality of surgical fasteners and a tissue cutting member. An anvil assembly is supported relative to the elongated channel. The surgical instrument further comprises a housing that operably supports at least a portion of a firing drive system that is configured to selectively generate firing motions. The housing further supports at least a portion of a closure system for generating closing and opening motions. The housing also supports at least a portion of an articulation system that is configured to generate articulation motions. An elongated shaft assembly are operably coupled to the housing and the surgical end effector and define a longitudinal tool axis. The elongated shaft assembly operably interface with the closure system to selectively apply the opening and closing motions to the end effector. The elongated shaft assembly also comprises a flexible articulation portion that includes a longitudinally-extending, elongated articulation spine that has a length and a width that that tapers along the length. The elongated articulation spine also has a component passage extending therethrough. A plurality of ribs extends from each lateral side of the articulation spine along a length thereof. Each rib include at least one anti-twist feature formed therein for preventing the flexible articulation portion from twisting about the longitudinal tool axis during articulation of the surgical end effector relative to the tool axis. The surgical instrument further comprises a flexible firing beam that is movably supported within the component passage and operably interfaces with the firing control system and the tissue cutting member. A right articulation beam is operably supported by the right ribs and operably interfaces with the articulation system and the elongate channel. A left articulation beam is operably supported by the left rib and interface with the articulation system and the elongate channel.

One skilled in the art will recognize that the herein described components (e.g., operations), devices, objects, and the discussion accompanying them are used as examples for the sake of conceptual clarity and that various configuration modifications are contemplated. Consequently, as used herein, the specific exemplars set forth and the accompanying discussion are intended to be representative of their more general classes. In general, use of any specific exemplar is intended to be representative of its class, and the non-inclusion of specific components (e.g., operations), devices, and objects should not be taken limiting.

With respect to the use of substantially any plural and/or singular terms herein, those having skill in the art translate from the plural to the singular and/or from the singular to the plural as is appropriate to the context and/or application. The various singular/plural permutations are not expressly set forth herein for sake of clarity.

The devices disclosed herein may be designed to be disposed of after a single use, or they may be designed to be used multiple times. In either case, however, the device may be reconditioned for reuse after at least one use. Reconditioning include any combination of the steps of disassembly of the device, followed by cleaning or replacement of particular pieces, and subsequent reassembly. In particular, the device is disassembled, and any number of the particular pieces or parts of the device are selectively replaced or removed in any combination. Upon cleaning and/or replacement of particular parts, the device is reassembled for subsequent use either at a reconditioning facility, or by a surgical team immediately prior to a surgical procedure. Those skilled in the art will appreciate that reconditioning of a device utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument then is sterilized. In one sterilization technique, the instrument is placed in a closed and sealed container, such as a plastic or TYVEK bag. The container and instrument are then placed in a field of radiation that penetrates the container, such as gamma radiation, x-rays, or high-energy electrons. The radiation kills bacteria on the instrument and in the container. The sterilized instrument then is stored in the sterile container. The sealed container keeps the instrument sterile until it is opened in the medical facility.

## Claims

1. A surgical instrument, comprising:
a surgical end effector (100); and
an elongated shaft assembly defining a longitudinal tool axis and being operably coupled to the surgical end effector (100), the elongated shaft assembly including a flexible segment (260) comprising:
a longitudinally extending, elongated articulation spine (262) including two lateral sides; and
a plurality of ribs (510, 520) extending laterally from each lateral side of said articulation spine (262) along a length thereof; wherein:
the two lateral sides of the articulation spine comprise:
a right lateral side; and
a left lateral side and wherein the plurality of ribs (510, 520) comprises:
at least three right ribs (510) protruding from the right lateral side of the articulation spine (262) such that first spaces (515) are provided between the right ribs (510), each of the first spaces (515) having a first space width (SWR); and
at least three left ribs (520) protruding from the left lateral side of the articulation spine (262) such that second spaces (525) are provided between the left ribs (520), each of the second spaces (525) having a second space width (SWL); **characterised in that**:
the spine (262) includes a length and a width that tapers along said length, wherein a distal portion of the flexible segment (260) articulates at an articulation rate that is faster than another articulation rate of another portion of the flexible segment (260) that is proximal to the distal portion upon application of articulation motions to the surgical end effector (100); and
the first space widths (SWR) sequentially decline in magnitude proceeding from a proximal end of the flexible segment (260) to a distal end of the flexible segment (260) and wherein the second space widths (SWL) sequentially decline in magnitude proceeding form the proximal end of the flexible segment (260) to the distal end of the flexible segment (260).

2. The surgical instrument of claim 1 wherein said articulation spine (262) includes a proximal end (264), a distal end (266) and a height and wherein said proximal end (264) includes a proximal width (PW) and wherein said distal end (266) includes a distal width (DW) that differs from said proximal width (PW).

3. The surgical instrument of claim 2 wherein said proximal width (PW) is greater than said distal width (DW).

4. The surgical instrument of claim 1 wherein at least one of the first space widths (SWR) differs from at least one other of the first space widths (SWR) and wherein at least one of the second space widths (SWL) differs from at least one other of the second space widths (SWL).

5. The surgical instrument of any preceding claim wherein at least one of the first space widths (SWR) is equal to at least one of the second space widths (SWL).

6. The surgical instrument of any preceding claim wherein at least two of said plurality of ribs (510) extending laterally on a right lateral side of said articulation spine (262) each include a right anti-twist protrusion (532) configured to engage a right anti-twist detent (534) in corresponding other said ribs (510) on said right lateral side and wherein at least two of said plurality of ribs (520) on a left lateral side of said articulation spine each (262) include a left anti-twist protrusion (532) configured to engage a left anti-twist detent (534) in corresponding other said ribs (520) on said left lateral side.

7. The surgical instrument of claim 6 wherein each said right anti-twist protrusion (532) and each said right anti-twist detent (534) are aligned along a right lateral axis (RLA-RLA) when said flexible portion (260) is unarticulated and wherein each said left anti-twist protrusion (532) and each said left anti-twist detent (534) are substantially aligned along a left lateral axis (LLA-LLA) when said flexible articulation portion (260) is unarticulated.

8. The surgical instrument of claim 1, wherein:
the end effector (100) comprises:
an elongated channel (102) configured to support a surgical fastener cartridge (110) therein that includes a plurality of surgical fasteners (120) and a tissue cutting member (150); and
an anvil (130) supported relative to said elongated channel (102) and wherein said surgical instrument further comprises:
a housing (20);
a firing drive system (300) configured to selectively generate firing motions wherein at least a portion of said firing drive system (300) is operably supported by said housing (20);
a closure system (200) for generating closing and opening motions and wherein at least a portion of said closure system is operably supported by said housing (20); and
an articulation system (400) configured to generate articulation motions wherein at least a portion of said articulation system (400) is operably supported by said housing (20); wherein
the elongated shaft assembly is operably coupled to said housing (20), said elongated shaft assembly operably interfacing with said closure system (200) to selectively apply said opening and closing motions to said end effector (100), wherein:
said elongated articulation spine (262) includes a component passage therethrough;
at least one said rib (510, 520) on each lateral side of said articulation spine (262) includes an anti-twist protrusion (532) that protrudes outward from the rib (510, 520) towards an adjacent said rib (510, 520) and wherein said adjacent said rib (510, 520) includes an anti-twist detent (534) that corresponds to said anti-twist protrusion (532) such that upon application of a twisting motion to said flexible articulation portion (260), said anti-twist protrusion (532) engages said corresponding anti-twist detent (534); said elongated shaft assembly further comprising:
a flexible firing beam (74) movably supported within said component passage and configured to operably interface with said firing drive system (300) and said tissue cutting member (150);
a right articulation beam operably supported by said right ribs (510) and operably interfacing with said articulation system (400) and said elongated channel; and
a left articulation beam operably supported by said left rib (520) and interfacing with said articulation system (400) and said elongated channel.

9. The surgical instrument of claim 8 further comprising:
a right sled pusher beam (76) movably supported in said component passage and interfacing with said firing drive system (300) and said surgical end effector (100); and
a left sled pusher beam (78) movably supported in said component passage and interfacing with said firing drive system (300) and said surgical end effector (100).

## Patentansprüche

1. Chirurgisches Instrument, umfassend:
einen chirurgischen Endeffektor (100); und
eine langgestreckte Schaftanordnung, die eine Werkzeuglängsachse definiert und mit dem chirurgischen Endeffektor (100) in funktionsfähiger Verbindung steht, wobei die langgestreckte Schaftanordnung ein flexibles Segment (260) aufweist, umfassend:
einen sich längs erstreckenden, länglichen Gelenkdorn (262) mit zwei lateralen Seiten; und
eine Vielzahl von Rippen (510, 520), die sich seitlich von jeder lateralen Seite des Gelenkdorns (262) entlang einer Länge davon erstrecken; wobei:
die zwei lateralen Seiten des Gelenkdorns das Folgende umfassen:
eine rechte laterale Seite; und
eine linke laterale Seite und wobei die Vielzahl von Rippen (510, 520) das Folgende umfasst:
mindestens drei rechte Rippen (510), die von der rechten lateralen Seite des Gelenkdorns (262) vorragen, so dass erste Zwischenräume (515) zwischen den rechten Rippen (510) bereitgestellt werden, wobei jeder der ersten Zwischenräume (515) eine erste Zwischenraumbreite (SWR) aufweist; und
mindestens drei linke Rippen (520), die von der linken lateralen Seite des Gelenkdorns (262) vorragen, so dass zweite Zwischenräume (525) zwischen den linken Rippen (520) bereitgestellt werden, wobei jeder der zweiten Zwischenräume (525) eine zweite Zwischenraumbreite (SWL) aufweist; **dadurch gekennzeichnet, dass**:
der Dorn (262) eine Länge und eine Breite aufweist, die sich entlang seiner Länge verjüngt;
wobei sich ein distaler Abschnitt des flexiblen Segments (260) mit einer Artikulationsrate gelenkig bewegt, die schneller als eine andere Artikulationsrate eines anderen Abschnitts des flexiblen Segments (260) ist, der proximal zum distalen Abschnitt ist, wenn der chirurgische Endeffektor (100) mit Gelenkbewegungen beaufschlagt wird; und
wobei die Größe der ersten Zwischenraumbreiten (SWR) von einem proximalen Ende des flexiblen Segments (260) zu einem distalen Ende des flexiblen Segments (260) sequentiell abnimmt und wobei die Größe der zweiten Zwischenraumbreiten (SWL) vom proximalen Ende des flexiblen Segments (260) zum distalen Ende des flexiblen Segments (260) sequentiell abnimmt.

2. Chirurgisches Instrument nach Anspruch 1, wobei der Gelenkdorn (262) ein proximales Ende (264), ein distales Ende (266) und eine Höhe aufweist und wobei das proximale Ende (264) eine proximale Breite (PW) aufweist und wobei das distale Ende (266) eine distale Breite (DW) aufweist, die sich von der proximalen Breite (PW) unterscheidet.

3. Chirurgisches Instrument nach Anspruch 2, wobei die proximale Breite (PW) größer als die distale Breite (DW) ist.

4. Chirurgisches Instrument nach Anspruch 1, wobei sich mindestens eine der ersten Zwischenraumbreiten (SWR) von mindestens einer anderen der ersten Zwischenraumbreiten (SWR) unterscheidet und wobei sich mindestens eine der zweiten Zwischenraumbreiten (SWL) von mindestens einer anderen der zweiten Zwischenraumbreiten (SWL) unterscheidet.

5. Chirurgisches Instrument nach einem der vorhergehenden Ansprüche, wobei mindestens eine der ersten Zwischenraumbreiten (SWR) mindestens einer der zweiten Zwischenraumbreiten (SWL) gleicht.

6. Chirurgisches Instrument nach einem der vorhergehenden Ansprüche, wobei sich mindestens zwei der Vielzahl von Rippen (510), die sich seitlich auf einer rechten lateralen Seite des Gelenkdorns (262) erstrecken, jeweils einen rechten verdrehungsfesten Vorsprung (532) aufweisen, der dazu ausgelegt ist, eine rechte Verdrehungssicherungssperre (534) in entsprechenden anderen Rippen (510) auf der rechten lateralen Seite in Eingriff zu nehmen und wobei mindestens zwei der Vielzahl von Rippen (520) auf einer linken lateralen Seite des Gelenkdorns (262) einen linken verdrehungsfesten Vorsprung (532) aufweisen, der dazu ausgelegt ist, eine linke Verdrehungssicherungssperre (534) in entsprechenden anderen Rippen (520) auf der linken lateralen Seite in Eingriff zu nehmen.

7. Chirurgisches Instrument nach Anspruch 6, wobei jeder rechte verdrehungsfeste Vorsprung (532) und jede rechte Verdrehungssicherungssperre (534) entlang einer rechten Seitenachse (RLA-RLA) ausgerichtet sind, wenn der flexible Abschnitt (260) nicht gelenkig bewegt wird, und wobei jeder linke verdrehungsfeste Vorsprung (532) und jede linke Verdrehungssicherungssperre (534) im Wesentlichen entlang einer linken Seitenachse (LLA-LLA) ausgerichtet sind, wenn der flexible Gelenkabschnitt (260) nicht gelenkig bewegt wird.

8. Chirurgisches Instrument nach Anspruch 1, wobei:
der Endeffektor (100) das Folgende umfasst:
einen langgestreckten Kanal (102), der zur Unterstützung einer chirurgischen Befestigungselementkassette (110) darin ausgelegt ist, die eine Vielzahl von chirurgischen Befestigungselementen (120) und ein Gewebeschneideelement (150) aufweist; und
einen Amboss (130), der bezüglich des langgestreckten Kanals (102) unterstützt wird, und wobei das chirurgische Instrument ferner das Folgende umfasst:
ein Gehäuse (20);
ein Abfeuerantriebssystem (300), das zur selektiven Erzeugung von Abfeuerbewegungen ausgelegt ist, wobei mindestens ein Teil des Abfeuerantriebssystems (300) vom Gehäuse (20) funktionsfähig unterstützt wird;
ein Schließsystem (200) zum Erzeugen von Schließ- und Öffnungsbewegungen und wobei mindestens ein Teil des Schließsystems vom Gehäuse (20) funktionsfähig unterstützt wird; und
ein Gelenksystem (400), das zum Erzeugen von Gelenkbewegungen ausgelegt ist, wobei mindestens ein Teil des Gelenksystems (400) vom Gehäuse (20) funktionsfähig unterstützt wird; wobei
die langgestreckte Schaftanordnung mit dem Gehäuse (20) in funktionsfähiger Verbindung steht, wobei die langgestreckte Schaftanordnung mit dem Schließsystem (200) in funktionsfähige Verbindung kommt, um den Endeffektor (100) selektiv mit den Öffnungs- und Schließbewegungen zu beaufschlagen; wobei:
der langgestreckte Gelenkdorn (262) einen Komponentendurchgang durch ihn hindurch aufweist;
mindestens eine Rippe (510, 520) auf jeder lateralen Seite des Gelenkdorns (262) einen verdrehungsfesten Vorsprung (532) aufweist, der von der Rippe (510, 520) nach außen zu einer benachbarten Rippe (510, 520) vorragt und wobei die benachbarte die Rippe (510, 520) eine Verdrehungssicherungssperre (534) aufweist, die dem verdrehungsfesten Vorsprung (532) entspricht, so dass bei Beaufschlagen des flexiblen Gelenkabschnitts (260) mit einer Verdrehbewegung der verdrehungsfeste Vorsprung (532) die entsprechende Verdrehungssicherungssperre (534) in Eingriff nimmt; wobei die langgestreckte Schaftanordnung ferner das Folgende umfasst:
einen flexiblen Abfeuerstab (74), der beweglich innerhalb des Komponentendurchgangs unterstützt wird und für eine funktionsfähige Verbindung mit dem Abfeuerantriebssystem (300) und dem Gewebeschneideelement (150) ausgelegt ist;
einen rechten Gelenkstab, der von den rechten Rippen (510) funktionsfähig unterstützt wird und mit dem Gelenksystem (400) und dem langgestreckten Kanal in funktionsfähiger Verbindung steht; und
einen linken Gelenkstab, der von der linken Rippe (520) funktionsfähig unterstützt wird und mit dem Gelenksystem (400) und dem langgestreckten Kanal in funktionsfähiger Verbindung steht.

9. Chirurgisches Instrument nach Anspruch 8, ferner umfassend:
einen rechten Schlittenschiebestab (76), der beweglich im Komponentendurchgang unterstützt wird und mit dem Abfeuerantriebssystem (300) und dem chirurgischen Endeffektor (100) in funktionsfähiger Verbindung steht; und
einen linken Schlittenschiebestab (78), der beweglich im Komponentendurchgang unterstützt wird und mit dem Abfeuerantriebssystem (300) und dem chirurgischen Endeffektor (100) in funktionsfähiger Verbindung steht.

## Revendications

1. Instrument chirurgical, comprenant :
un effecteur terminal chirurgical (100) ; et
un ensemble d'arbre allongé définissant un axe d'outil longitudinal et étant accouplé fonctionnellement à l'effecteur terminal chirurgical (100), l'ensemble d'arbre allongé comportant un segment flexible (260) comprenant :
une épine d'articulation allongée (262), s'étendant longitudinalement, comportant deux côtés latéraux ; et
une pluralité de nervures (510, 520) s'étendant latéralement depuis chaque côté latéral de ladite épine d'articulation (262) le long d'une longueur de celle-ci ;
les deux côtés latéraux de l'épine d'articulation comprenant :
un côté latéral droit ; et
un côté latéral gauche et la pluralité de nervures (510, 520) comprenant :
au moins trois nervures droites (510) faisant saillie depuis le côté latéral droit de l'épine d'articulation (262) de telle sorte que des premiers espaces (515) soient prévus entre les nervures droites (510), chacun des premiers espaces (515) ayant une première largeur d'espace (SWR) ; et
au moins trois nervures gauches (520) faisant saillie depuis le côté latéral gauche de l'épine d'articulation (262) de telle sorte que des deuxièmes espaces (525) soient prévus entre les nervures gauches (520), chacun des deuxièmes espaces (525) ayant une deuxième largeur d'espace (SWL) ;
**caractérisé en ce que** :
l'épine (262) présente une longueur et une largeur qui s'effilent le long de ladite longueur ;
une portion distale du segment flexible (260) s'articulant avec une vitesse d'articulation qui est supérieure à une autre vitesse d'articulation d'une autre portion du segment flexible (260) qui est située en position proximale par rapport à la portion distale lors de l'application de mouvements d'articulation à l'effecteur terminal chirurgical (100) ; et
les premières largeurs d'espace (SWR) diminuant séquentiellement en amplitude à partir d'une extrémité proximale du segment flexible (260) jusqu'à une extrémité distale du segment flexible (260) et les deuxièmes largeurs d'espace (SWL) diminuant séquentiellement en amplitude à partir de l'extrémité proximale du segment flexible (260) jusqu'à l'extrémité distale du segment flexible (260) .

2. Instrument chirurgical selon la revendication 1, dans lequel ladite épine d'articulation (262) comporte une extrémité proximale (264), une extrémité distale (266) et une hauteur et dans lequel ladite extrémité proximale (264) comporte une largeur proximale (PW) et ladite extrémité distale (266) comporte une largeur distale (DW) différente de ladite largeur proximale (PW).

3. Instrument chirurgical selon la revendication 2, dans lequel ladite largeur proximale (PW) est supérieure à ladite largeur distale (DW).

4. Instrument chirurgical selon la revendication 1, dans lequel au moins l'une des premières largeurs d'espace (SWR) est différente d'au moins une autre des premières largeurs d'espace (SWR) et dans lequel au moins l'une des deuxièmes largeurs d'espace (SWL) est différente d'au moins une autre des deuxièmes largeurs d'espace (SWL).

5. Instrument chirurgical selon l'une quelconque des revendications précédentes, dans lequel au moins l'une des premières largeurs d'espace (SWR) est égale à au moins l'une des deuxièmes largeurs d'espace (SWL).

6. Instrument chirurgical selon l'une quelconque des revendications précédentes, dans lequel au moins deux de ladite pluralité de nervures (510) s'étendant latéralement sur un côté latéral droit de ladite épine d'articulation (262) comportent chacune une saillie droite anti-torsion (532) configurée pour venir en prise avec un ergot droit anti-torsion (534) dans d'autres desdites nervures (510) correspondantes sur ledit côté latéral droit et dans lequel au moins deux de ladite pluralité de nervures (520) sur un côté latéral gauche de ladite épine d'articulation comportent chacune (262) une saillie gauche anti-torsion (532) configurée pour venir en prise avec un ergot gauche anti-torsion (534) dans d'autres desdites nervures (520) correspondantes sur ledit côté latéral gauche.

7. Instrument chirurgical selon la revendication 6, dans lequel chaque dite saillie droite anti-torsion (532) et chaque dit ergot droit anti-torsion (534) sont alignés le long d'un axe latéral droit (RLA-RLA) lorsque ladite portion flexible (260) n'est pas articulée et dans lequel chaque dite saillie gauche anti-torsion (532) et chaque dit ergot gauche anti-torsion (534) sont sensiblement alignés le long d'un axe latéral gauche (LLA-LLA) lorsque ladite portion d'articulation flexible (260) n'est pas articulée.

8. Instrument chirurgical selon la revendication 1, dans lequel :
l'effecteur terminal (100) comprend :
un canal allongé (102) configuré pour supporter une cartouche d'agrafes chirurgicales (110) dans celui-ci, qui comporte une pluralité d'agrafes chirurgicales (120) et un organe de découpe des tissus (150) ; et
une enclume (130) supportée par rapport audit canal allongé (102) et dans lequel ledit instrument chirurgical comprend en outre :
un boîtier (20) ;
un système d'entraînement de tir (300) configuré pour générer de manière sélective des mouvements de tir, dans lequel au moins une portion dudit système d'entraînement de tir (300) est supportée fonctionnellement par ledit boîtier (20) ;
un système de fermeture (200) pour générer les mouvements de fermeture et d'ouverture et dans lequel au moins une portion dudit système de fermeture est supportée fonctionnellement par ledit boîtier (20) ; et
un système d'articulation (400) configuré pour générer des mouvements d'articulation, dans lequel au moins une portion dudit système d'articulation (400) est supportée fonctionnellement par ledit boîtier (20) ;
l'ensemble d'arbre allongé étant accouplé fonctionnellement audit boîtier (20), ledit ensemble d'arbre allongé réalisant fonctionnellement une interface avec ledit système de fermeture (200) pour appliquer de manière sélective lesdits mouvements d'ouverture et de fermeture audit effecteur terminal (100),
ladite épine d'articulation allongée (262) comportant un passage de composant à travers elle ;
au moins une dite nervure (510, 520) sur chaque côté latéral de ladite épine d'articulation (262) comportant une saillie anti-torsion (532) qui fait saillie vers l'extérieur depuis la nervure (510, 520) vers une dite nervure adjacente (510, 520), et ladite nervure dite adjacente (510, 520) comportant un ergot anti-torsion (534) qui correspond à ladite saillie anti-torsion (532) de telle sorte que lors de l'application d'un mouvement de torsion à ladite portion d'articulation flexible (260), ladite saillie anti-torsion (532) vienne en prise avec ledit ergot anti-torsion correspondant (534), ledit ensemble d'arbre allongé comprenant en outre :
une barre de tir flexible (74) supportée de manière déplaçable à l'intérieur dudit passage de composant et configurée pour réaliser fonctionnellement une interface avec ledit système d'entraînement de tir (300) et avec ledit organe de découpe des tissus (150) ;
une barre d'articulation droite supportée fonctionnellement par lesdites nervures droites (510) et réalisant fonctionnellement une interface avec ledit système d'articulation (400) et ledit canal allongé ; et
une barre d'articulation gauche supportée fonctionnellement par ladite nervure gauche (520) et réalisant une interface avec ledit système d'articulation (400) et ledit canal allongé.

9. Instrument chirurgical selon la revendication 8, comprenant en outre :
une barre de poussoir de chariot droite (76) supportée de manière déplaçable dans ledit passage de composant et réalisant une interface avec ledit système d'entraînement de tir (300) et ledit effecteur terminal chirurgical (100) ; et
une barre de poussoir de chariot gauche (78) supportée de manière déplaçable dans ledit passage de composant et réalisant une interface avec ledit système d'entraînement de tir (300) et ledit effecteur terminal chirurgical (100).
